(19) **Europäisches Patentamt**
**European Patent Office**
**Office européen des brevets**

(11) **EP 4 038 179 B1**

(12) **EUROPEAN PATENT SPECIFICATION**

(45) Date of publication and mention
of the grant of the patent:
**22.04.2026  Bulletin 2026/17**

(21) Application number: **20788864.5**

(22) Date of filing: **02.10.2020**

(51) International Patent Classification (IPC):
**C12N 5/0775** $^{(2010.01)}$    **A61K 35/28** $^{(2015.01)}$

(52) Cooperative Patent Classification (CPC):
**C12N 5/0663; A61K 35/28; C12N 2500/38;**
C12N 2501/115; C12N 2501/15; C12N 2501/39

(86) International application number:
**PCT/GB2020/052439**

(87) International publication number:
**WO 2021/064424 (08.04.2021 Gazette 2021/14)**

(54) **BONE MARROW MESENCHYMAL STEM CELL DERIVED CELL POPULATIONS AND METHODS OF PREPARING SAME**

AUS KNOCHENMARK STAMMENDE MESENCHYMALE STAMMZELLPOPULATIONEN UND VERFAHREN ZUR HERSTELLUNG DERSELBEN

POPULATIONS DE CELLULES DÉRIVÉES DE CELLULES SOUCHES MÉSENCHYMATEUSES DE MOELLE OSSEUSE ET LEURS PROCÉDÉS DE PRÉPARATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **02.10.2019  GB 201914235**

(43) Date of publication of application:
**10.08.2022  Bulletin 2022/32**

(73) Proprietor: **THE UNIVERSITY OF LIVERPOOL**
**Liverpool L69 7ZX (GB)**

(72) Inventor: **HOLLANDER, Anthony Peter**
**Liverpool Merseyside L69 7ZX (GB)**

(74) Representative: **HGF**
**HGF Limited**
**4th Floor, 1 City Square**
**Leeds LS1 2ES (GB)**

(56) References cited:
**WO-A1-2013/067038    WO-A2-2019/035668**
**US-B2- 8 431 162**

• **SALINAS ET AL: "The enhancement of chondrogenic differentiation of human mesenchymal stem cells by enzymatically regulated RGD functionalities", BIOMATERIALS, ELSEVIER, AMSTERDAM, NL, vol. 29, no. 15, 4 March 2008 (2008-03-04), pages 2370 - 2377, XP022526909, ISSN: 0142-9612, DOI: 10.1016/ J.BIOMATERIALS.2008.01.035**
• **YUEH-HSUN KEVIN YANG ET AL: "Changes in phenotype and differentiation potential of human mesenchymal stem cells aging in vitro", STEM CELL RESEARCH & THERAPY, vol. 9, no. 1, 11 May 2018 (2018-05-11), XP055757373, DOI: 10.1186/s13287-018-0876-3**
• **VICTORIA CHAPMAN ET AL: "Therapeutic Benefit for Late, but Not Early, Passage Mesenchymal Stem Cells on Pain Behaviour in an Animal Model of Osteoarthritis", STEM CELLS INTERNATIONAL, vol. 2017, 1 January 2017 (2017-01-01), US, pages 1 - 11, XP055757624, ISSN: 1687-966X, DOI: 10.1155/2017/2905104**

**(Cont. next page)**

- MIQI WANG ET AL: "Trophic stimulation of articular chondrocytes by late-passage mesenchymal stem cells in coculture : LATE-PASSAGE MESENCHYMAL STEM CELL COCULTURE", JOURNAL OF ORTHOPAEDIC RESEARCH, vol. 31, no. 12, 29 August 2013 (2013-08-29), US, pages 1936 - 1942, XP055757626, ISSN: 0736-0266, DOI: 10.1002/jor.22466

- ANNA SALERNO ET AL: "MMP13 and TIMP1 are functional markers for two different potential modes of action by mesenchymal stem/stromal cells when treating osteoarthritis", STEM CELLS (MIAMISBURG), vol. 38, no. 11, 1 November 2020 (2020-11-01), pages 1438 - 1453, XP055757406, ISSN: 1066-5099, DOI: 10.1002/stem.3255

## Description

### FIELD OF THE INVENTION

The present disclosure relates to populations of cells that may be used as medicaments. The disclosure also relates to methods of preparing cells for use as medicaments. The disclosure further relates to methods for selecting mesenchymal stem cell (MSC)-derived cells for therapeutic uses, and to methods of treatment using MSC-derived cells.

### BACKGROUND

MSCs have been proposed as therapeutic agents for use in a wide range of applications, including tissue repair and regeneration, and amelioration of conditions such a graft-versus-host disease. The means by which MSCs achieve their therapeutic effects in vivo are incompletely understood, but are generally believe to include differentiation to produce new cells that contribute to replacement tissues, trophic effects that stimulate repair by neighbouring cells, and suppression of the local immune response.

One factor that has limited the uptake of MSC-based treatments is the ability to generate therapeutically effective numbers of cells. Many treatments require large numbers of cells, that are most effectively produced by long periods of cell culture. However, it is known that properties associated with the therapeutic uses of MSCs can change significantly over time in culture. For example, the capacity of MSCs to differentiate into multiple phenotypes (multipotency), a key characteristic of stem cells, is gradually lost during prolonged cell culture, while other therapeutically relevant properties may be retained. Accordingly, prolonged culture, of the sort desirable to produce large numbers of cells, appears to be incompatible with the retention of properties that contribute to the therapeutic activities of MSCs *in vivo,* or with the generation of homogenous populations of the sort needed for robust cell-based medicines.

YUEH-HSUN KEVIN YANG ET AL: "Changes in phenotype and differentiationpotential of human mesenchymal stem cells aging in vitro", STEM CELL RESEARCH & THERAPY, vol. 9, no. 1, 11 May 2018 (2018-05-11), DOI: 10.1186/s13287-018-0876-3 discusses how Human MSCs subjected to extensive in-vitro passage can undergo morphological, phenotypic and genetic changes.

US8431162B2 (ZUBA-SURMA EWA K [US]; DAWN BUDDHADEB [US] ET AL.) 30 April 2013 (2013-04-30) describes an isolated sub-population of bone marrow-derived adherent cells.

WO 2013/067038 A1 (NEOSTEM INC [US]; MARASCO WAYNE [US] ET AL.) 10 May 2013 (2013-05-10) discusses a composition comprising MPCs and processes for isolating or enriching the MPCs.

WO 2019/035668 A2 (SUNGKWANG MEDICAL FOUND) 21 February 2019 (2019-02-21) discusses a composition comprising mesenchymal stem cells as an effective ingredient for prevention, alleviation, or treatment of thyroid associated opthalmopathy.

VICTORIA CHAPMAN ET AL: "Therapeutic Benefit for Late, but Not Early, Passage Mesenchymal Stem Cells on Pain Behaviour in an Animal Model of Osteoarthritis", STEM CELLS INTERNATIONAL, vol. 2017, 1 January 2017 (2017-01-01), pages 1-11, us ISSN: 1687-966X, DOI: 10.1155/2017/2905104 discusses the role of passage number in influencing the therapeutic effects of MSCs in a model of osteoarthritic pain.

MIQI WANG ET AL: "Trophic stimulation of articular chondrocytes by late-passage mesenchymal stem cells in coculture: LATE-PASSAGE MESENCHYMAL STEM CELL COCULTURE", JOURNAL OF ORTHOPAEDIC RESEARCH, vol. 31, no. 12, 29 August 2013 (2013-08-29), pages 1936-1942, US ISSN: 0736-0266, DOI: 10.1002/jor. 22466 discusses that coculture of MSCs with ACs increases glycosaminoglycan accumulation compared to monoculture.

SALINAS ET AL: "The enhancement of chondrogenic differentiation of human mesenchymal stem cells by enzymatically regulated RGD functionalities", BIOMATERIALS, ELSEVIER, AMSTERDAM, NL, vol. 29, no. 15, 4 March 2008 (2008-03-04), pages 2370-2377, XP022526909, ISSN: 0142-9612, DOI: 10.1016/ J.BIOMATERIALS.2008.01.035 discusses that temporal regulation of integrin-binding peptides is important in the design of niches in differentiating hMSCs to chondrocytes.

### SUMMARY OF THE INVENTION

According to a first aspect of the invention, there is provided a method of selecting an MSC-derived cell population for therapeutic use, the method comprising:

- assessing levels of MMP13 gene expression and TIMP-1 protein secretion by a cell population, and
- selecting a cell population for use in a therapeutic application that does not require a capacity for phenotypic differentiation if the cell population exhibits low levels of MMP13 gene expression defined by an average level of MMP13 expression relative to a housekeeping gene that is half or less than half of said relative expression by freshly isolated MSCs, and high levels of TIMP-1 protein secretion defined by an average secretion of 100ng/ml or more of TIMP-1 from cells initially plated at a density of 2.25 million cells/well in a 24 hour period, wherein the therapeutic application that does not require a capacity for phenotypic differentiation is selected from the group consisting of: promoting tissue repair via trophic activity; and promotion of therapeutic immunosuppression.

In one embodiment the cell population is derived from MSCs by passaging of MSCs.

According to a second aspect of the invention, there is provided a pharmaceutical composition comprising a population of cells enriched for an MSC-derived cell population , wherein said MSC-derived cell population is :

- CD90+; CD105+; CD45-
- High TIMP-1 secretion defined by an average secretion of 100ng/ml or more of TIMP-1 from cells initially plated at a density of 2.25 million cells/well in a 24 hour period,
- Low MMP13 gene expression defined by an average level of MMP13 expression relative to a housekeeping gene that is half or less than half of said relative expression by freshly isolated MSCs, and Has trophic activity,
- wherein the population of cells enriched for an MSC-derived cell population comprises at least 70% of cells having the recited marker profile,

and a pharmaceutically acceptable excipient.

In some embodiments of the second aspect the population of cells enriched for an MSC-derived cell population comprises at least 70% of cells having the recited marker profile.

In some embodiments of the second aspect the population of cells enriched for an MSC-derived cell population comprises at least 80% of cells that are positive for CD90 and CD105, and 10% or less of cells that are positive for CD45.

In some embodiments of the second aspect the pharmaceutical composition is substantially free from cells that are: CD90+; CD105+; CD45-; and have high TIMP-1 secretion and high MMP13 gene expression. Preferably the pharmaceutical composition is free from cells that are: CD90+; CD105+; CD45-; and have high TIMP-1 secretion and high MMP13 gene expression.

Also disclosed herein is a population of cells enriched for MSC-derived cells that are:

- CD90[+]; CD105[+]; CD45[-]
- High TIMP-1 secretion and
- Low MMP13 gene expression

for use as a medicament.

The population of cells for medical use defined herein are able to stimulate trophic repair, as discussed in more detail elsewhere in the specification. A population of cells of the disclosure may be free from (or substantially free from) cells that are: CD90[+]; CD105[+]; CD45[-]; and have high TIMP-1 secretion and high MMP13 gene expression.

The medicament may be used in promoting tissue repair, and/or in promoting therapeutic immunosuppression.

Also disclosed herein is a method of preparing cells for use as a medicament, the method comprising:

- culturing MSCs to produce MSC-derived cells;
- enriching the MSC-derived cells for a population that are:

  - CD90[+]; CD105[+]; CD45[-]
  - High TIMP-1 protein secretion and
  - Low MMP13 gene expression; and

- formulating the cells for use as a medicament.

The medicament produced by formulation of cells prepared by the method of the disclosure may be referred to as "a medicament of the disclosure". A medicament of the disclosure may be free from (or substantially free from) cells that are: CD90[+]; CD105[+]; CD45[-]; and have high TIMP-1 secretion and high MMP13 gene expression.

Also disclosed herein is a method of selecting an MSC-derived cell for therapeutic use, the method comprising:

- assessing levels of MMP13 gene expression and TIMP-1 protein secretion by a cell, and

- selecting a cell for use in a therapeutic application that does not require a capacity for phenotypic differentiation if the cell exhibits low levels of MMP13 gene expression and high levels of TIMP-1 protein secretion.

In a suitable example, a method in accordance with the disclosure may optionally further comprise a step of selecting a cell for use in a therapeutic application requiring a capacity for phenotypic differentiation if the cell exhibits high levels of both MMP13 gene expression and TIMP-1 protein secretion.

It will be appreciated that the methods of the dislcosure may be put into practice by selecting populations of cells that have the desired characteristics. Examples of suitable techniques, including technique for enrichment of the desired cell types, are described elsewhere in this disclosure.

Also disclosed herein is a method of treatment, the method comprising providing a therapeutically effective amount of cells that are:

- CD90$^+$; CD105$^+$; CD45$^-$
- High TIMP-1 protein secretion and
- Low MMP13 gene expression;

to a subject in need of treatment.

The subject may be one in need of tissue repair, and/or in need of therapeutic immunosuppression.

Also disclosed herein is a pharmaceutical composition comprising a population of cells enriched for MSC-derived cells that are:

- CD90$^+$; CD105$^+$; CD45$^-$
- High TIMP-1 secretion and
- Low MMP13 gene expression

and a pharmaceutically acceptable excipient.

A pharmaceutical composition of the disclosure may be free from (or substantially free from) cells that are: CD90$^+$; CD105$^+$; CD45$^-$; and have high TIMP-1 secretion and high MMP13 gene expression

## BRIEF DESCRIPTION OF THE DRAWINGS

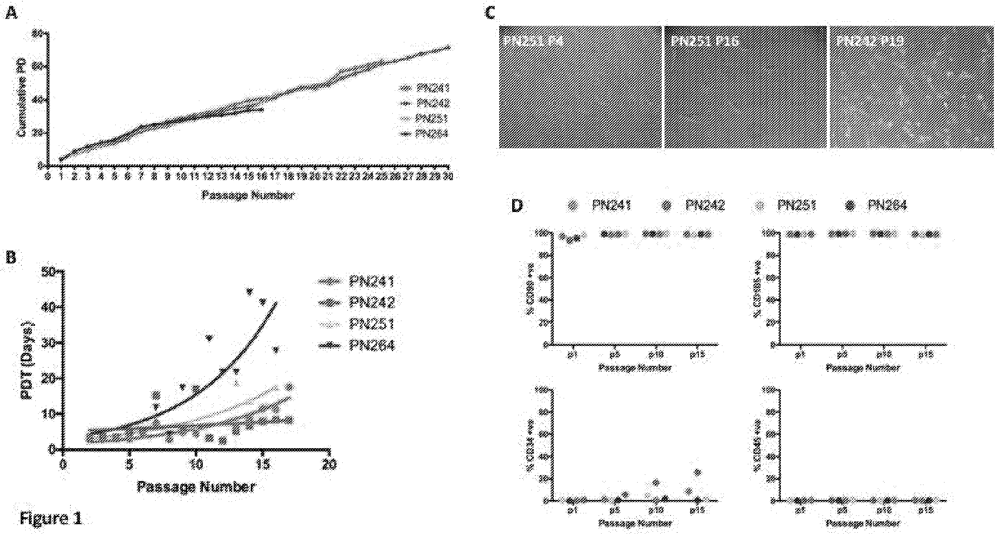

*Figure 1. Growth and phenotypic characteristics of MSCs from four patients*
(A) The number of populations doublings reached after each passage using MSCs from each patient were recorded until growth ceased (for PN242 data were collected until passage 30, when the cells continued to grow). (B) The population doubling time is shown up to passage 17 for all four patients. (C) Typical cell morphology is shown for PN251 MSCs at passage 4 and passage 16 (growth arrest was at passage 17 for this patient) and for PN242 at passage 19 (no growth arrest observed for this patient). Size bars = 200$\mu$M. (D) The percentage of cells expressing MSC markers CD90 and CD105 and haematopoietic stem cell markers CD34 and CD45 were determined by Fluorescence Activated Cell Sorting. As discussed further elsewhere, the cells of the disclosure demonstrate a characteristic combination of being CD90 and CD105 positive, and CD45 negative.

*Figure 2. Macroscopic appearance of tissue engineered cartilage made using MSCs from four patients at multiple passages*
MSCs from passages 2-16 were seeded onto polyglycolic acid scaffolds before being induced to undergo chondrogenic differentiation. Each image shows replicate samples from each patient at each passage that was investigated.

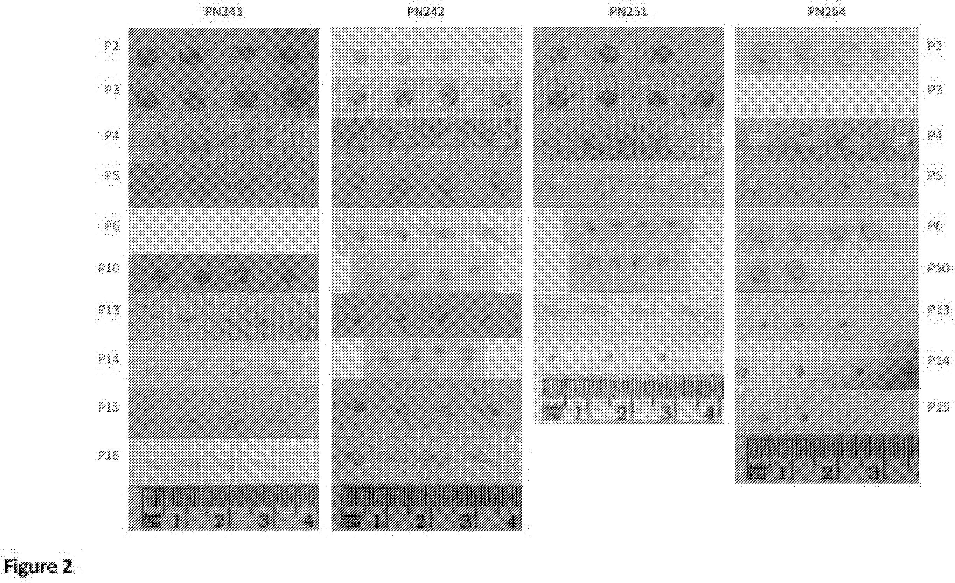

*Figure 3. Biochemical properties of tissue engineered cartilage made using MSCs from four patients at multiple passages*
MSCs from each patient across a range of passages were used to tissue engineer cartilage, as shown in Figure 2. (A-D) The relationship between dry weight or glycosaminoglycans (GAG) content of tissue engineered cartilage constructs and the population doublings of MSCs at the time of tissue engineering are shown for each of the patients. For all the graphs, each point shows the mean value of multiple tissue engineering replicates made using cells from one patient at a single passage. (E) The relationship between dry weight of tissue engineered cartilage constructs and the passage number of MSCs at the time of tissue engineering are shown for all four patients. Each point is the result for a single replicate sample. (F) The relationship between the GAG content of tissue engineered cartilage constructs and the passage number of MSCs at the time of tissue engineering are shown for all four patients. Each point is the result for a single replicate sample. For all graphs, the line represents a linear model of degree 1 fitted to the points whilst the spearman rank correlation coefficient and its significance is shown in the top right corner.

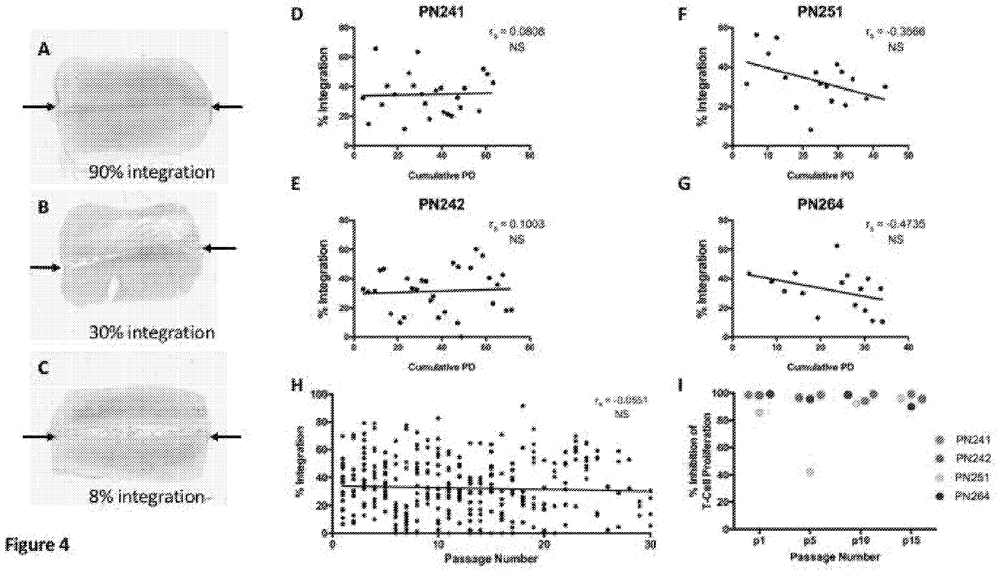

*Figure 4. Trophic repair of meniscal cartilage and immunoregulation by MSCs from four patients at multiple*

*passages*

For trophic repair of meniscal cartilage, undifferentiated MSCs were seeded onto a collagen scaffolds, inserted between two pieces of sheep meniscus, clipped together and cultured for 30 days. Histomorphometry was used to measure the degree of integration between the two pieces of meniscal tissue. (A-C) Typical examples of high, medium and poor integration of meniscal tissue. Integration was measured in between the arrows. Size bars = 1mm. (D-G) The relationship between % integration of meniscal tissue and the population doublings of MSCs are shown for each of the patients. For all the graphs, each point shows the mean value of multiple experimental replicates using cells from one patient at a single passage. (H) The relationship between % integration of meniscal tissue and the passage number of MSCs are shown for all four patients. Each point is the result for a single replicate sample. For all correlation graphs (A-H), the line represents a linear model of degree 1 fitted to the points whilst the spearman rank correlation coefficient and its significance is shown in the top right corner. (I) Immunoregulation was determined as the % inhibition of T-cell proliferation, measured as the loss of Cell-Trace Violet from labelled cells, quantified by FACS. Each point is the result for pooled triplicate wells.

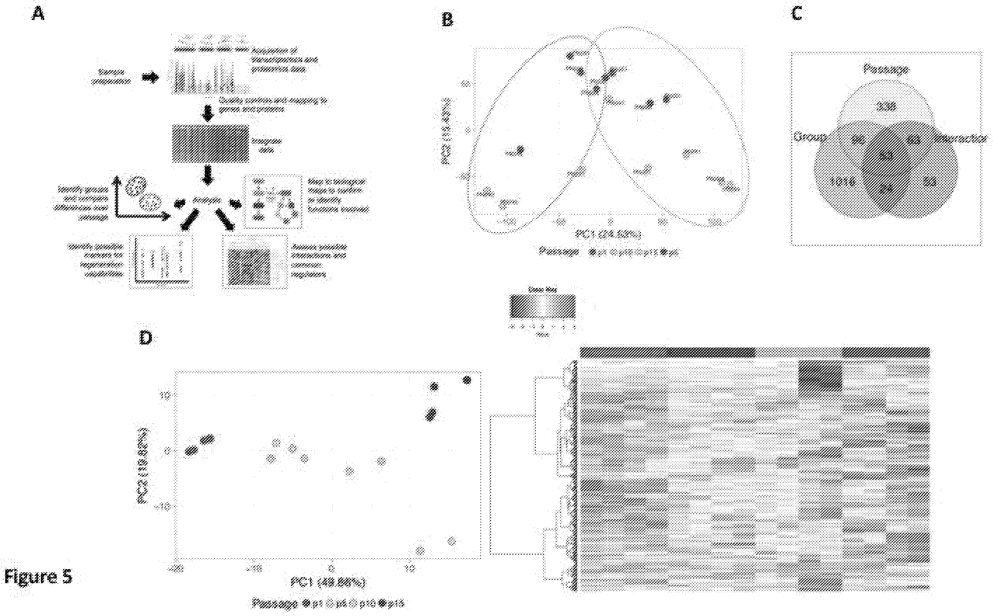

### Figure 5. Transcriptomics and proteomics

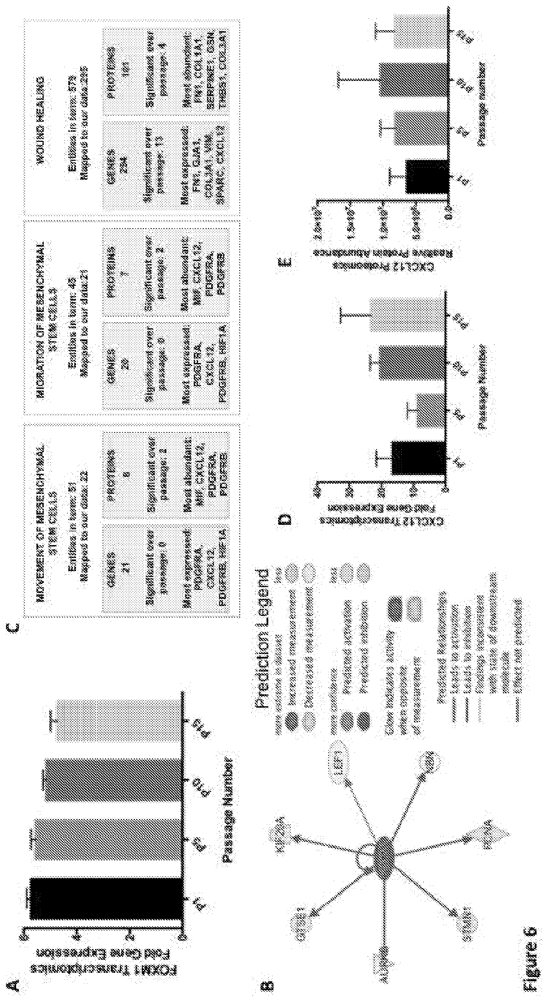

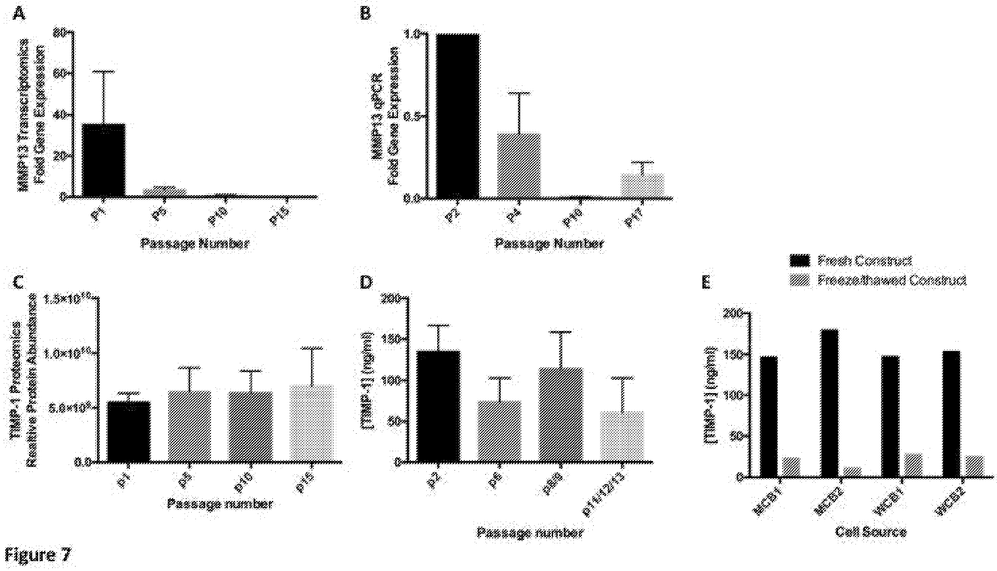

(A) Overview of analytical steps performed. Transcriptomics and proteomics data were processed according to data standards and integrated. The integrated data were further analysed by Ingenuity Pathway Analysis to identify key genes and proteins that change over passage and map them to biological functions and predict possible upstream regulators (see **Figures 6 and 7).** (B) Principal Component Analysis (PCA) score plot of first two principal components of all genes and proteins. There is a segregation of patients (see blue and orange ellipses) that can also be linked to changes in osteogenic capabilities (see Figures S2), whilst changes related to passage are capture mainly in PC2. (C) Venn diagram of significant variables after 2-way ANOVA test revealing 338 genes and proteins that vary significantly and independently of the patient group variation. (D) PCA of the 338 significant variables over passage. Shown the score plot of the first 2 principal components (capturing approximately 70% of variance). As expected there is a marked segregation over passage. Heatmap of scaled data from the 338 significant proteins and genes that are represented in rows and clustered via Ward hierarchical clustering. Columns represent biological samples, each column is a patient sample and they are ordered by passage with red being passage 1, green passage 5, turquoise passage 10 and purple passage 15.

### Figure 6. Ingenuity Pathway Analysis of integrated transcriptomics and proteomics data

(A) Ingenuity pathway analysis identified FOXM1 and four other master regulators genes (see **Figure S5)** that vary with increasing passage number. FOXM1 data from transcriptomics analysis shows inhibition of gene expression with increasing passage number. Each bar is the mean $\pm$ SEM for results using MSCs from each of the four patients. (B) Ingenuity pathway analysis of the integrated transcriptomics and proteomics data set predicts inhibition of five out of six of the identified downstream regulators in the FOXM1 canonical pathway. (C) Ingenuity pathway analysis identified multiple genes and proteins that mapped to the search terms "Cell Movement", Cell Migration" or "Wound Healing". Most of these identified genes and proteins did not vary significantly over passage as determined by ANOVA. The most highly expressed genes and proteins are listed for each of the search terms. (D) CXCL12 data from transcriptomics analysis shows continuous gene expression with increasing passage number. Each bar is the mean $\pm$ SEM for results using MSCs from each of the four patients. (E) CXCL12 data from proteomics analysis shows continuous protein secretion with increasing passage number. Each bar is the mean $\pm$ SEM for results using MSCs from each of the four patients.

### Figure 7. Gene and protein markers of the in vitro MSC ageing process

The MMP13 gene was selected as a marker of early passage cells which is lost with ageing of MSCs *in vitro* whilst secretion of the TIMP-1 protein was selected as a marker of MSCs that is independent of *in vitro* ageing. (A) MMP13 data from transcriptomics analysis shows decreasing gene expression with increasing passage number. Each bar is the mean $\pm$ SEM for results using MSCs from each of the four patients. (B) MMP13 data from qPCR analysis shows decreasing gene expression with increasing passage number. Each bar is the mean $\pm$ SEM for results using MSCs from each of the four patients. (C) TIMP-1 data from proteomics analysis shows continuous protein secretion with increasing passage number. Each bar is the mean $\pm$ SEM for results using MSCs from each of the four patients. (D) TIMP-1 data from ELISA analysis shows continuous protein secretion with increasing passage number. Each bar is the mean $\pm$ SEM for results using MSCs from each of the four patients. (E) ELISA analysis of TIMP-1 secreted by MSC/collagen scaffold constructs shows continuous protein secretion with increasing passage number for fresh constructs but reduced secretion from constructs that have been freeze-thawed under conditions that reduce their viability. This pattern of high TIMP-1 secretion and low MMP13 gene expression is characteristic of the cells of the disclosure. In particular, it (in combination with the CD90 positive, CD105 positive, CD45 negative expression profile discussed elsewhere) it is indicative of cells suitable for use in therapeutic applications that do not require a capacity for phenotypic differentiation. Such applications may make use of trophic activity or therapeutic immunosuppressive activity of the cells instead. For each cells source the result is shown for one fresh compared with one frozen construct.

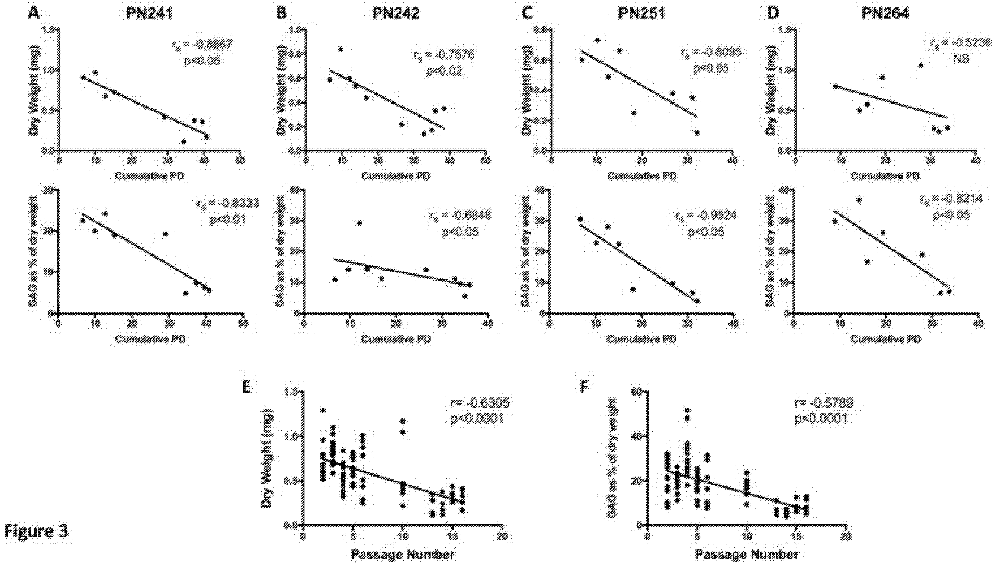

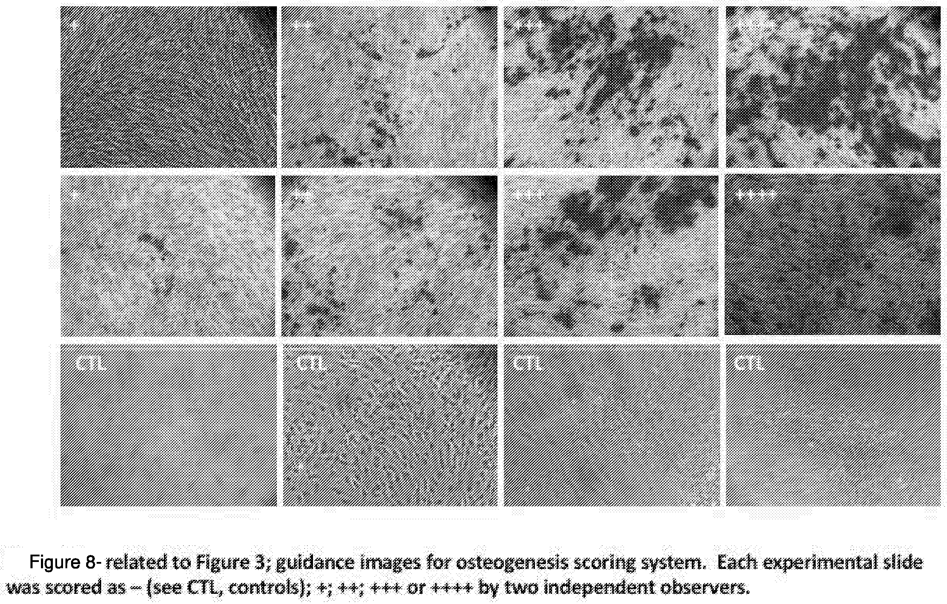

***Figure 8 (related to Figure 3). Guidance template images for osteogenic scoring***
MSCs were induced to undergo osteogenic differentiation and then stained with alizarin red. Each slide was scored as - (see CTL, controls); +; ++; +++ or ++++ by two independent observers. Size bars = 200$\mu$M.

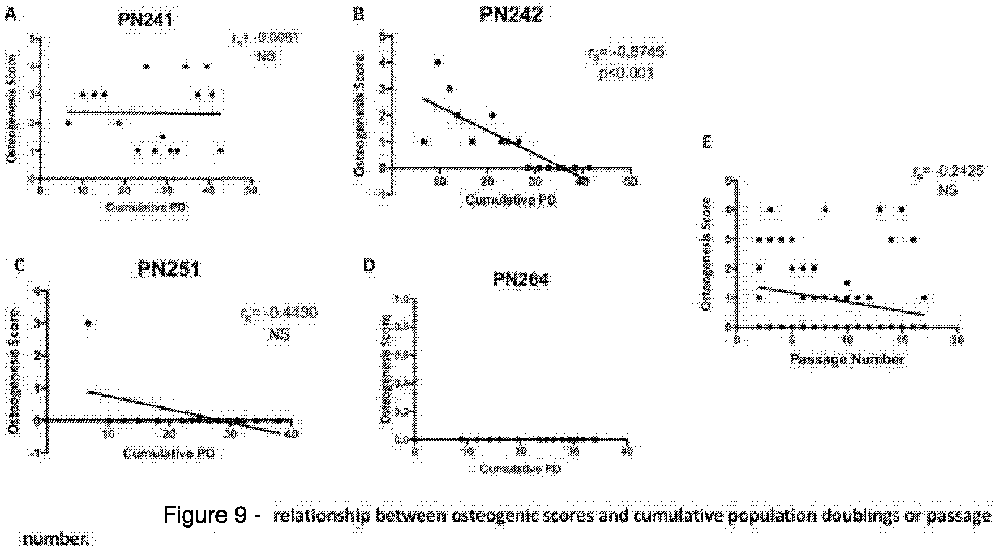

***Figure 9 (related to Figure 3). Osteogenic differentiation of MSCs from four patients at multiple passages***
MSCs from each patient across a range of passages were induced to undergo osteogenic differentiation *in vitro* and scored as shown in Figure 8. (A-D) The relationship between osteogenic score and the cumulative population doublings of MSCs are shown for each of the patients. For all the graphs, each point shows the mean value of multiple replicates using cells from one patient at a single passage. (E) The relationship between the osteogenic score and the passage number of MSCs are shown for all four patients. Each point is the result for a single replicate sample. For all graphs, the line represents a linear model of degree 1 fitted to the points whilst the spearman rank correlation coefficient and its significance is shown in the top right corner.

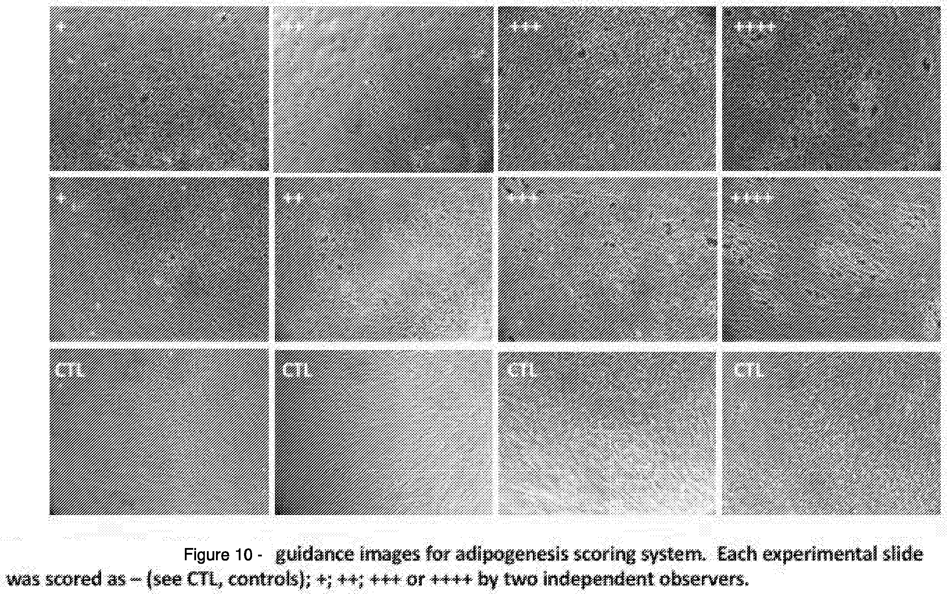

***Figure 10 (related to Figures 3). Guidance template images for adipogenic scoring***
MSCs were induced to undergo adipogenic differentiation and then stained with oil red O. Each slide was scored as - (see CTL, controls); +; ++; +++ or ++++ by two independent observers. Size bars = 200$\mu$M.

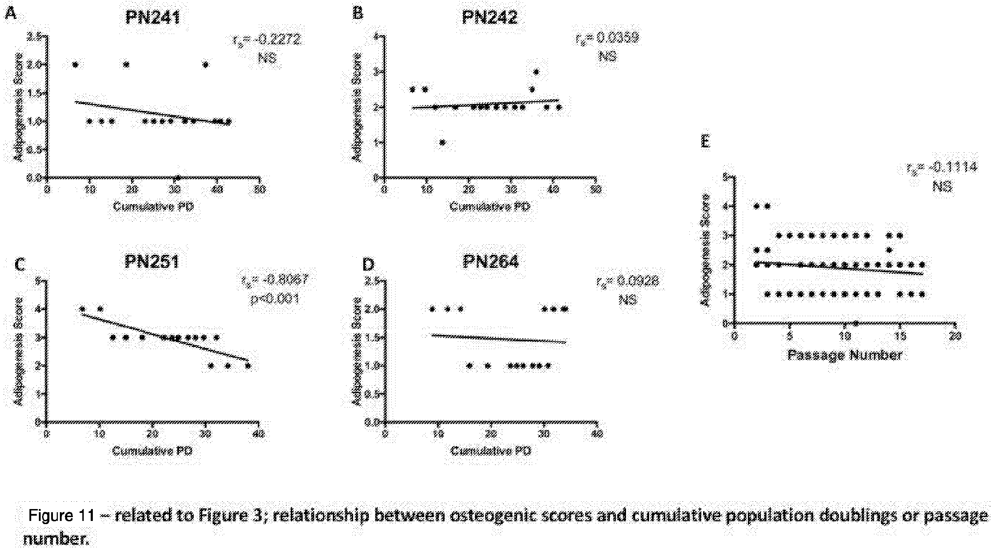

***Figure 11 (related to Figure 3). Adipogenic differentiation of MSCs from four patients at multiple passages***
MSCs from each patient across a range of passages were induced to undergo adipogenic differentiation *in vitro* and scored as shown in Figure S3. (A-D) The relationship between adipogenic score and the cumulative population doublings of MSCs are shown for each of the patients. For all the graphs, each point shows the mean value of multiple replicates using cells from one patient at a single passage. (E) The relationship between the adipogenic score and the passage number of MSCs are shown for all four patients. Each point is the result for a single replicate sample. For all graphs, the line represents a linear model of degree 1 fitted to the points whilst the spearman rank correlation coefficient and its significance is shown in the top right corner.

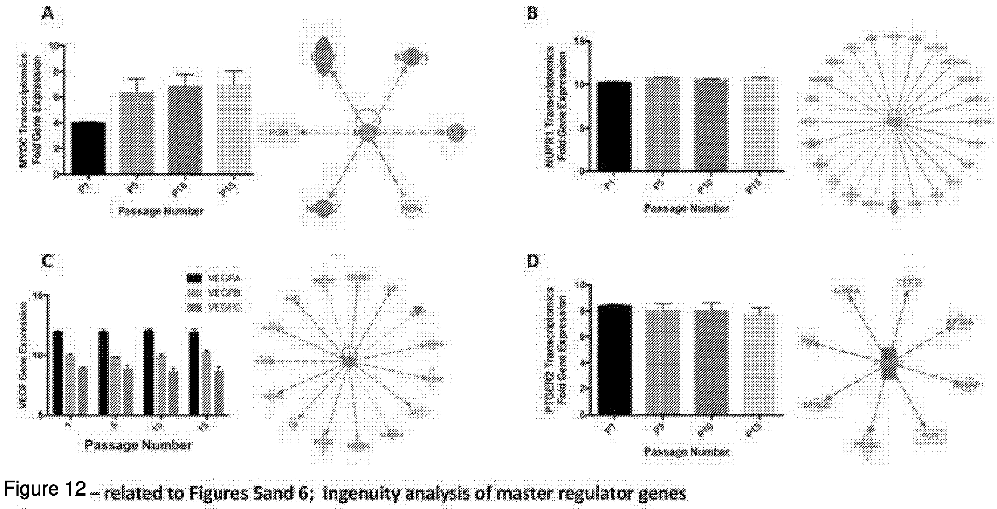

***Figure 12 (related to Figures 5 and 6). Ingenuity Pathway Analysis of genes and proteins that vary with increasing passage***
Ingenuity pathway analysis identified FOXM1 (See Figure 6) and four other master regulators genes that vary with increasing passage number. (A) Relationship between MYOC data from transcriptomics analysis and increasing passage number and Ingenuity pathway analysis of the integrated transcriptomics and proteomics data set. Each bar is the mean $\pm$ SEM for results using MSCs from each of the four patients. (B) Relationship between NUPR1 data from transcriptomics analysis and increasing passage number and Ingenuity pathway analysis of the integrated transcriptomics and proteomics data set. Each bar is the mean $\pm$ SEM for results using MSCs from each of the four patients. (C) Relationship between VEGF data from transcriptomics analysis and increasing passage number and Ingenuity pathway analysis of the integrated transcriptomics and proteomics data set. Each bar is the mean $\pm$ SEM for results using MSCs from each of the four patients. (D) Relationship between PTGER2 data from transcriptomics analysis and increasing passage number and Ingenuity pathway analysis of the integrated transcriptomics and proteomics data set. Each bar is the mean $\pm$ SEM for results using MSCs from each of the four patients.

## DETAILED DESCRIPTION OF THE INVENTION

The present invention is based, at least in part, upon the inventors' finding, disclosed for the first time in this document, that MSCs cultured *in vitro* exhibit a selective loss of multipotent potential, but not trophic activity or immunosuppressive activity, prior to senescence. This finding, and the observation that many of the therapeutic uses of MSCs and MSC-derived cells arise as a consequence of the trophic or immunosuppressive activity of such cells, rather than through phenotypic differentiation of these cells, opens a new possibility of providing therapeutic uses of cultured cells that would previously have been discounted from such applications. That extensively cultured cells can be used therapeutically in this manner confers a significant benefit, in that it allows the generation of much larger numbers of therapeutically useful cells than would previously have been thought possible.

Furthermore, the inventors have identified a characteristic panel of markers that allows those cells that retain trophic or immunosuppressive activity, but are not capable of phenotypic differentiation, to be distinguished from those cells that retain capacity for phenotypic differentiation (either with or without trophic or immunosuppressive activity). This characteristic expression profile (cells that are: CD90+; CD105+; CD45-; have high TIMP-1 secretion and low MMP13 gene expression) has not been identified before. Its identification here provides significant advantages for medical use of MSC-derived cells in practice.

It will be recognised that retained trophic or immunosuppressive activity, but absence of phenotypic differentiation potential, on the part of the populations of cells for medical use disclosed herein ensures that these cells are able to achieve their therapeutic activity without giving rise to new cell populations at damaged areas. This is advantageous as it reduces or avoids the potential for undesirable outcomes that may potentially be associated with *in situ* differentiation of MSCs or MSC-derived cells (an advantage shared by other aspects of the invention).

Furthermore, it has previously been recognised that the therapeutic properties of cultured MSCs (or MSC-derived cells) change with time. Cells that have been grown for longer periods of time (or for higher passage numbers) may be able to alleviate pain in damaged joints, while cells at earlier passage numbers may exacerbate such pain. A given group of cells may contain populations of cells at various different stages in this process, and so it is important to be able to distinguish between these populations, and to select only those required for a desired therapeutic use (particularly the more "aged" populations).

While differences in the properties of cultured MSCs have previously been recognised, there has not, prior to the disclosure of the present invention, been a reliable way in which to determine that the "switch" to cells or populations of cells with therapeutic trophic or immunosuppressive activity, but without complicating or undesirable properties (such as potential for phenotypic differentiation or exacerbation of joint pain), has occurred. By identifying the importance of the marker profile CD90+; CD105+; CD45-; high TIMP-1 secretion and low MMP13 gene expression, the inventors have enabled reliable and robust selection of these advantageous cells and populations.

The identification of this marker profile avoids the need for selection based upon criteria, such as passage number, that are associated with significant failings. Merely by way of example, these include the impact of variations among individuals in the point at which this change occurs (relevant for both allogeneic and autologous cell sources), variation in rates of cell division within cell cultures, and reliance on considerations, such as passage number, that are not relevant in the case of modern bioreactor-based culture conditions.

The populations of cells enriched for MSC-derived cells that are CD90+; CD105+; CD45-; have high TIMP-1 secretion and low MMP13 gene expression, represent cell populations that have increased homogeneity, which is important in the ability to deliver predictable and reproducible cell-based medicaments in practice.

It will be appreciated from the results disclosed herein that discrimination between cell populations on the basis of low or high MMP13 gene expression is particularly important in allowing a distinction to be drawn between populations of cells that have trophic or immunosuppressive therapeutic activity, but which lack capacity for phenotypic differentiation, and populations of cells that retain the ability for phenotypic differentiation (such as chondrogenesis). Such cells may be CD90+; CD105+; CD45-; and have high TIMP-1 secretion and high MMP13 gene expression, in contrast to the cells of the disclosure (that are CD90+; CD105+; CD45-; and have high TIMP-1 secretion and low MMP13 gene expression).

In view of the above, it will be recognised that the present disclosure identifies for the first time that:

- trophic repair and immunoregulation are retained in prolonged culture;
- MSC-derived cells retain the ability to exert these therapeutic mechanisms after extensive cell expansion; and
- provides means by which cells having these desired characteristics can be selected from those having less beneficial properties (such as retained capacity for phenotypic differentiation).

MSCs are used for a wide range of clinical applications for tissue regeneration (e.g. bone and cartilage; cardiovascular disease) as well as for disease modification, including haematological disease, graft-versus-host disease and inflammatory diseases. Some of these clinical approaches are based on differentiation whilst others depend on trophic or immunoregulatory functions.

There have been several studies describing the loss of differentiation capacity with increasing passage of MSCs *in vitro*, with other studies suggesting that *in vivo* ageing also leads to a loss of differentiation capacity after *ex vivo* isolation of the aged cells. These observations combined with parallel observations of shortening in telomere length with *in vitro* ageing of MSCs have been interpreted as indicating a rapid senescence process that compromises cell function and limits their clinical utility.

Having found for the first time that cells derived from MSCs that have been extensively passaged retain their trophic and immunosuppressive activities, even when the potential for multipotent differentiation is lost, the inventors have recognised that such cells are still able to provide valuable therapeutic effects. Furthermore, they have identified a characteristic pattern of markers that allow these therapeutically effective cells to be recognised, selected, and incorporated into medicaments after undergoing cell culture.

The disclosure will now be described further with reference to the following paragraphs.

## Populations of cells

For the present purposes, the considerations below may be taken as applicable to populations of cells according to the disclosure , as well as to populations of cells selected in the methods of the disclosure, or for use in the methods of treatment of the disclosure.

Cells of the disclosure may be autologous cells, or may be allogeneic cells. The cells of the disclosure may not have the capacity for multipotent differentiation. The skilled person will be aware of many methods by which multipotency, or absence of multipotency, may be determined, including (but not limited to) those discussed in the Examples.

## Characterisation of cells with respect to cell markers

The therapeutically useful MSC-derived cells disclosed herein exhibit a characteristic expression pattern in respect of a number of markers. These include cell surface markers (CD90, CD105, and CD45) and functional markers (TIMP-1 and MMP13).

## Cell surface markers

### CD90

CD90, also known as Thy-1, is an N-glycosylated glycophosphatidylinositol (GPI) anchored cell surface protein that has a single V-like immunoglobulin domain. As discussed further below, the therapeutically useful MSC-derived cells described in this disclosure are characteristically CD90$^+$.

### CD105

CD105, also known as endoglin, is a type I membrane glycoprotein. It is located on the cell surface, where it functions as part of the TGF-$\beta$ receptor complex. As discussed further below, the therapeutically useful MSC-derived cells described in this disclosure are characteristically CD105$^+$.

### CD45

CD45, also known as protein tyrosine phosphatase receptor type C (PTPRC), is a type I transmembrane protein involved in a number of cell signalling pathways. As discussed further below, the therapeutically useful MSC-derived cells described in this disclosure are characteristically CD45$^-$.

## Characterisation of cells as positive or negative for cell surface markers

The expression (or absence of expression) of cell surface markers including CD90, CD105 and CD45 by populations of cells may be investigated by any suitable means known to those skilled in the art. Merely by way of example, cell surface markers may be labelled and detected by appropriate reagents such as antibodies specific for the cell markers of interest. Antibody labelling approaches of this sort can be used in combination with cell selection techniques of the sorts further described below.

A cell may be considered positive ("+ve" or "+") for a particular cell surface marker (such as CD90 or CD105) if, after appropriate immunofluorescence labelling, it has a level of fluorescence that is greater than 90% of cells treated with corresponding control antibodies, such as isotype-matched control antibodies.

A cell may be considered negative ("-ve" or "-") for a particular cell surface marker (such as CD45) if, after appropriate immunofluorescence labelling, it has a level of fluorescence that is less than 10% of cells treated with corresponding control antibodies, such as isotype-matched control antibodies.

## Functional cell markers

### TIMP-1

TIMP-1, also known as TIMP metallopeptidase inhibitor 1, is a member of the tissue inhibitor of metallopeptidases family. It is a glycoprotein that functions as an inhibitor of matrix metalloproteinases. As discussed further below, the therapeutically useful MSC-derived cells described in this disclosure characteristically have high TIMP-1 protein secretion.

### MMP13

MMP13 (matrix metallopeptidase 13, also known as collagenase 3) is a metalloproteinase enzyme involved in the breakdown of extracellular membrane components. It is encoded by the *MMP13* gene in humans. As discussed further below, the therapeutically useful MSC-derived cells described in this disclosure characteristically have low MMP13 gene expression.

The relevance of this marker in allowing the selection of cell populations that have trophic and immunosuppressive activity, but not the capacity for phenotypic differentiation, has not previously been reported. As reported herein, the inventors have found that this marker is key in distinguishing cells disclosed herein (which are: CD90⁺; CD105⁺; CD45⁻; and have high TIMP-1 secretion and low MMP13 gene expression) from other populations of cells that do not offer the same therapeutic advantages, but retain capacity for phenotypic differentiation (which are: CD90⁺; CD105⁺; CD45⁻; and have high TIMP-1 secretion and high MMP13 gene expression).

**Characterisation of cells as high or low for functional cell markers**

The cells disclosed herein are characterised as having high TIMP-1 protein secretion.

Expression and secretion of TIMP-1 may be investigated using any suitable technique for the assessment of protein secretion known to those skilled in the art. Merely by way of example, TIMP-1 protein expression may be investigated using an enzyme linked immunoassay (ELISA) approach. Examples of an appropriate ELISA for TIMP-1 secretion that may be used to determine whether or not cells of interest are positive for TIMP-1 secretion in the manner required of cells disclosed herein is described further in the Examples.

The cells disclosed herein are characterised as having low MMP13 gene expression.

MMP13 gene expression may be assessed by investigating levels of transcripts indicative of MMP13 expression within a cell. Suitably MMP13 gene expression may be assessed by MMP13 specific reverse transcription PCR. A particularly suitable method based upon this technique is described further in the Examples.

A population of cells may be identified as having high TIMP-1 secretion on the basis of the quantity of this protein secreted by a set number of cells over a set period of time. Suitably high secretion of TIMP-1 protein may be indicated by the secretion of 100ng/ml (or more) of TIMP-1 from cells (initially plated at a density of 2.25 million cells/well) in a 24 hour period. Further details of suitable conditions that may be used in such an assessment are set out in the Examples, in connection with the study reported in Figure 7E.

A population of cells may be identified as having low MMP13 gene expression on the basis of comparison to a suitable baseline. Merely by way of example, a housekeeping gene such as Tata binding protein may provide a suitable baseline. A population of cells may be characterised as having low MMP13 gene expression if these cells demonstrate a level of MMP13 expression relative to a housekeeping gene (such as Tata binding protein) that is half or less than half of the relative expression by freshly isolated MSCs. Suitable freshly isolated MSCs for comparison purposes may be derived from a source that is appropriately matched to that of the cells disclosed herein. Comparison of folds of expression (whether of MMP13 or suitable housekeeping genes) may be carried out using the methods described further in the Examples.

*Enrichment of cells and enriched populations of cells*

A population of cells disclosed herein is enriched for the presence of therapeutically useful MSC-derived cells, as compared to naturally occurring cell populations. Cells prepared in a method disclosed herein, are also enriched for the presence of such cells, and the selection of cells disclosed herein may give rise to enriched populations of cells in the same manner.

The therapeutically useful MSC-derived cells to be enriched within a population may be identified with reference to their characteristic expression of markers (i.e. CD90⁺, CD105⁺, CD45⁻; high TIMP-1 protein secretion; and low MMP13 gene expression).

The skilled person will understand the term "enriched" as used in the context of cell populations. Enrichment may be achieved by positive selection of desired cells (i.e. those with the characteristic expression of markers) or by elimination of undesired cells.

Merely by way of example, enrichment approaches may make use of culture conditions that favour the proliferation of cells with desired traits (such as the characteristic expression of markers discussed herein), and/or conditions that diminish the viability of cells that lack desired traits. Cells grown in such conditions may be monitored to ensure that the desired population are enriched. Merely by way of example, cell culture conditions that can be used to enrich populations of therapeutically effective MSC-derived cells disclosed herein may include the use of 10% foetal calf serum, and supplementation with 5ng/ml fibroblast growth factor (FGF2).

Alternatively, enrichment or selection may be understood in the context of techniques such as fluorescence activated cell sorting (FACS) that allow the selection of cells on the basis of their expression of markers (such as CD90, CD105, and CD45). Unwanted cells may then be discarded.

Thus, a method of preparing cells for use as a medicament disclosed herein may comprise a step of selecting cells with reference to one or more of the recited markers (CD90$^+$; CD105$^+$; CD45$^-$; high TIMP-1 secretion; and low MMP13 gene expression), and thus enriching the MSC-derived cells for the specified population of cells. A suitable selection step may comprise selection based on low MMP13 gene expression, a marker that the inventors have found to be particularly useful in identifying cells that retain trophic activity without potential for phenotypic differentiation.

Suitably an enriched population of MSC-derived cells may comprise at least 80% of cells that are positive for CD90 and CD105, and 10% or less of cells that are positive for CD45.

For the purposes of the present disclosure, an enriched population of cells comprises at least 70% cells having the recited characteristic marker profile (i.e. CD90$^+$; CD105$^+$; CD45$^-$; high TIMP-1 secretion; and low MMP13 gene expression). Suitably an enriched population of cells may comprise at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% cells having the recited characteristic marker profile. An enriched population of cells may consist of substantially 100% cells having the recited characteristic marker profile (i.e. CD90$^+$; CD105$^+$; CD45$^-$; high TIMP-1 secretion; and low MMP13 gene expression).

A population of cells disclosed herein, or selected in a method disclosed herein, may substantially consist entirely of cells having the recited characteristic marker profile (i.e. CD90$^+$; CD105$^+$; CD45$^-$; high TIMP-1 secretion; and low MMP13 gene expression).

A population of cells disclosed herein, or selected in a method disclosed herein, may be free, or substantially free, from cells which are: CD90$^+$; CD105$^+$; CD45$^-$; and have high TIMP-1 secretion and high MMP13 gene expression.

Cell enrichment or cell selection may be practiced by any suitable method, including, but not limited to, those discussed herein.

### Cell selection and analysis

Having been informed of the characteristic marker profile of cells disclosed herein, the skilled person would be able to readily identify methods by which cells exhibiting this profile may be selected. Similarly, the skilled person would readily identify methods by which populations of cells may be investigated to determine whether they exhibit characteristic expression patterns. Without limitation, these include techniques selected from the group consisting of: flow cytometry; fluorescence activated cell sorting (FACS); and magnetic activated cell sorting (MACS).

### Cell culture

The inventors have found that the trophic and immunosuppressive activities of cells disclosed herein are retained through prolonged cell culture. This has the advantage of allowing very large numbers of therapeutically effective cells to be generated. As set out elsewhere in the specification, cells disclosed herein may retain therapeutically effective levels of trophic or immunoregulatory activity for up to 30 passages.

Accordingly, a population of cells disclosed herein may comprise cells that have been subject to at least 5 passages in culture, at least 10 passages in culture, at least 15 passages in culture, at least 20 passages in culture, at least 25 passages in culture, or at least 30 passages in culture.

Similarly, a method disclosed herein may comprise culturing the cells for at least 5 passages in culture, at least 10 passages in culture, at least 15 passages in culture, at least 20 passages in culture, at least 25 passages in culture, or at least 30 passages in culture.

### Medical uses and methods of treatment

The populations of cells disclosed herein, the medicaments produced from cells prepared as disclosed herein, the cells selected by the methods disclosed herein, and the methods of treatment disclosed herein are all suitable for therapeutic use. In particular, the therapeutic use may be a use to promote tissue repair, or use to promote therapeutic immunosuppression. Further details of these examples are set out below.

### Tissue repair

Medicaments, medical uses or methods of treatment disclosed herein may be used to promote tissue repair. The requirement for promotion of tissue repair may arise as a result of any condition causing tissue injury. For example, the requirement for promotion of tissue repair may arise as a result of a condition selected from the group consisting of: osteoarthritis; myocardial infarction; meniscus cartilage injury (such as torn meniscus); ligament injury (such as torn ligament); injuries to the skin; and soft tissue injury. The tissue repair may be promoted by promoting therapeutic trophic activity. The tissue repair may ameliorate pathology associated with a diseased or damaged joint. This may be assessed by appropriate means, such as histological examination (of biopsies or experimentally treated joints) or suitable imaging techniques.

Suitably the tissue repair may comprise treatment of a damaged tissue to promote tissue repair by improving the rate of tissue repair and/or improving the quality of repaired tissue that results from treatment. The promotion of tissue repair may be readily assessed with reference to a suitable control.

The cells, methods of treatment or medical uses disclosed herein may be capable of promoting tissue repair by at least 10% as compared to suitable controls. Merely by way of example, the cells, methods of treatment or medical uses disclosed herein may be capable of promoting tissue repair by at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95%, as compared to suitable controls. Indeed, the cells, methods of treatment or medical uses disclosed herein may be capable of promoting tissue repair by 100% or more as compared to suitable controls.

Suitably the tissue repair may comprise treatment of a damaged tissue selected from the group consisting of: cartilage; cardiovascular tissue; bone; and soft tissue, such as skin.

Suitably, the tissue repair may comprise treatment of a condition selected from the group consisting of: treatment of torn cartilage, such as treatment of torn meniscal cartilage; treatment of: osteoarthritis; myocardial infarction; meniscus cartilage injury (such as torn meniscus); ligament injury (such as torn ligament); injuries to the skin; and soft tissue injury.

It will be appreciated that trophic activity of MSC-derived cells may contribute to tissue repair. Accordingly a medical use or method of treatment employing a population of cells or medicament disclosed herein, may be for use in tissue repair by the stimulation of trophic activity. Such uses may be contrasted with those applications that have previously been described in which MSCs or MSC-derived cells achieve their therapeutic utility via phenotypic differentiation (i.e. through differentiation to yield replacement cells that contribute to tissue repair).

### Trophic activity

The cells disclosed herein have trophic activity that is retained from the MSCs from which they are derived. The medical uses and methods of treatment disclosed herein, particularly those for use in tissue repair, may make use of the trophic activity of such cells. Indeed cells disclosed herein may be selected for their trophic activity (and absence of capacity for phenotypic differentiation).

Trophic activity may be considered as the capacity by which, following implantation, MSCs or MSC-derived cells are able to induce neighbouring cells to secrete active molecules that contribute to tissue repair or regeneration. Trophic repair may occur as a result of the production by the MSCs, or MSC-derived cells, of large amounts of growth factors and other mediators. Trophic activity has been shown to contribute to tissue repair or regeneration in the treatment of stroke, myocardial infarction, and meniscal cartilage repair, among other examples.

The methods disclosed herein may further comprise a step of assessing the cells to investigate their trophic activity. Suitably cells to be used as a medicament may be selected based upon their trophic activity. The populations of cells disclosed herein may be enriched for MSC-derived cells that have trophic activity.

The presence or absence of trophic activity may be assessed by any suitable methods known to those skilled in the art. Suitable methods may investigate the ability of cells to ameliorate pathology of diseased or damaged joints. Merely by way of example, the trophic activity of cells, such as cells disclosed herein, or prepared in a method disclosed herein, may be assessed by testing their capacity to integrate separate regions of cartilage. Suitably the capacity to promote such integration may be demonstrated *in vitro*. Alternatively, or additionally, such capacity may be demonstrated *in vivo*.

Details of an assay that allows assessment of trophic activity via integration of separate regions of cartilage are provided in "Repair of torn avascular meniscal cartilage using undifferentiated autologous mesenchymal stem cells: from *in vitro* optimization to a first-inhuman study" (Whitehouse, et al., Stem Cells Translation Medicine, 2017; 6:1237-1248), insofar as it relates to the model for investigating integration of cartilage treated with test agents. Details of a suitable technique are also set out in the Examples of the present disclosure.

Therapeutic applications that can benefit from the trophic activity of cells or medicaments, or medical uses or methods of treatment, disclosed herein correspond to those that will benefit from promotion of tissue repair.

### Therapeutic immunosuppression

The cells of the disclosure also have immunosuppressive activity that is retained from the MSCs from which they are derived. The medical uses and methods of treatment disclosed herein may make use of the cells' immunosuppressive activity, and may be for use in therapeutic immunosuppression.

There are a number of therapeutic applications in which it is known to utilise the immunosuppressive properties of MSCs, or MSC-derived cells. Suitably, the therapeutic immunosuppression may be employed in the treatment of a disease selected from the group consisting of: haematological disease; graft-versus-host disease; and alloreactive immune disease, autoimmune disease, or inflammatory disease.

Therapeutic immunosuppression using the cells, methods of treatment or medical uses of the disclosure may be utilised in the treatment of patients that have received stem cell grafts, such as grafts of haematopoietic stem cells. Patients receiving such grafts may be suffering from a haematological disease.

The ability of the cells, methods of treatment and medical uses of the disclosure to achieve therapeutically effective immunosuppressive activity is also of use in the treatment of graft-versus-host disease. In such examples, treatment with cells or medicaments of the disclosure may be able to reduce the recipient's immune response, and thus alleviate the response to an allogeneic graft.

There are also a number of conditions in which disease initiation or progression is mediated by the body's immune response. These include alloreactive immune diseases, autoimmune diseases, or inflammatory diseases. Such diseases may benefit from treatment with the cells or medicaments of the disclosure, and by the medical uses or methods of treatment of the disclosure.

In a suitable example, a cell, medicament or method of treatment is accordance with the disclosure is for use in treatment of a disease by immunosuppression of the immune response.

### Immunosuppressive activity

The methods disclosed herein may further comprise a step of assessing the cells to investigate their immunosuppressive activity. Suitably cells to be used as a medicament may be selected based upon their immunosuppressive activity. The populations of cells in accordance with the disclosure may be enriched for MSC-derived cells that have immunosuppressive activity.

The presence or absence of immunosuppressive activity may be assessed by any suitable methods known to those skilled in the art. Immunosuppressive activity of cells or a medicament of the disclosure may be compared to that of a suitable control. The cells or medicaments of the disclosure may be able to achieve at least a 10% suppression of immune activity as compared to controls, when assessed in a suitable assay. The cells or medicaments of the disclosure may, for example be able to achieve at least a 15%, at least a 20%, at least a 25%, at least a 30%, at least a 35%, at least a 40%, at least a 45%, at least a 50%, at least a 55%, at least a 60%, at least a 65%, at least a 70%, at least a 75%, at least a 80%, at least a 85%, at least a 90%, or at least a 95% suppression of immune activity as compared to suitable controls. Indeed, the cells or medicaments of the disclosure may be able to achieve 100% suppression of immune activity as compared to suitable controls.

Merely by way of example, the immunosuppressive activity of cells, such as cells prepared in a method disclosed herein, may be assessed by testing their capacity to inhibit T-cell proliferation. In a suitable example, immunosuppressive activity is indicated by the ability to inhibit T-cell proliferation by at least 80%, consistent with the results set out in Figure 4I.

### Therapeutic applications that do or do not require a capacity for phenotypic differentiation

The disclosure provides a method by which suitable MSC-derived cells can be selected for therapeutic applications. This selection is undertaken with reference to the nature of the therapeutic application, and also the expression of certain markers by the MSC-derived cells.

The methods disclosed herein allow a distinction to be made between cells to be used in a therapeutic application that requires a capacity for phenotypic differentiation, and cells that are to be used in a therapeutic application that does not require a capacity for phenotypic differentiation.

Therapeutic applications that require a capacity for phenotypic differentiation will be those in which the therapeutic activity of MSC-derived cells is achieved by the ability of these cells to differentiate and generated new cells that provide a therapeutic effect. Examples of such applications include *in vitro* tissue engineering, in which MSCs are induced to differentiate and thereby to produce articular cartilage or bone, and the use of MSCs for transplantation into cartilage lesions or bone defects for the *in situ* generation of replacement tissues.

In contrast, there are a number of therapeutic applications that do not require MSC-derived cells to have a capacity for phenotypic differentiation in order to achieve a therapeutic effect. It will be appreciated that therapeutic applications that make use of trophic effects to induce tissue repair do not require a capacity for phenotypic differentiation, since it is trophic factors produced by MSCs or MSC-derived cells that mediate therapeutic activity of this sort. Similarly, it will be recognised that therapeutic applications that make use of the immunosuppressive effects of MSC-derived cells do not require a capacity for phenotypic differentiation. Examples of therapeutic applications that make use of trophic effects or immunosuppressive effects to achieve the required therapeutic activity are described elsewhere in the present specification. The populations of cells, cells, and methods of treatment disclosed herein are also highly suitable for use in such applications that do not require a capacity for phenotypic differentiation or multipotency.

## Formulations of cells

Populations of cells disclosed herein may be formulated as a pharmaceutical composition. Indeed, the disclosure also provides compositions including the cells disclosed herein, including pharmaceutical compositions and formulations, such as unit dose form compositions including the number of cells for administration in a given dose or fraction thereof. The pharmaceutical compositions and formulations generally include one or more optional pharmaceutically acceptable carrier or excipient. In some examples, the composition includes at least one additional therapeutic agent.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some examples, the choice of carrier is determined in part by the particular cell and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001 to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some cases are included in the compositions. Suitable buffering agents include, for example, citric acid, sodium citrate, phosphoric acid, potassium phosphate, and various other acids and salts. In some cases, a mixture of two or more buffering agents is used. The buffering agent or mixtures thereof are typically present in an amount of about 0.001 to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulations can include aqueous solutions. The formulation or composition may also contain more than one active ingredient useful for the particular indication, disease, or condition being treated with the cells, preferably those with activities complementary to the cells, where the respective activities do not adversely affect one another. Such active ingredients are suitably present in combination in amounts that are effective for the purpose intended. Thus, in some examples, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, and/or vincristine.

The pharmaceutical composition in some examples contains the cells in amounts effective to treat or prevent the disease or condition, such as a therapeutically effective or prophylactically effective amount. Therapeutic or prophylactic efficacy in some examples is monitored by periodic assessment of treated subjects. The desired dosage can be delivered by a single bolus administration of the cells, by multiple bolus administrations of the cells, or by continuous infusion administration of the cells.

The cells and compositions may be administered using standard administration techniques, formulations, and/or devices. Administration of the cells can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some examples, the cell populations are administered parenterally. The term "parenteral," as used herein, includes intravenous, intramuscular, subcutaneous, rectal, vaginal, and intraperitoneal administration. In some examples, the cells are administered to the subject using peripheral systemic delivery by intravenous, intraperitoneal, or subcutaneous injection.

Compositions in some examples are provided as sterile liquid preparations, e.g., isotonic aqueous solutions, suspensions, emulsions, dispersions, or viscous compositions, which may in some cases be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the cells in a solvent, such as in admixture with a suitable carrier, diluent, or excipient such as sterile water, physiological saline, glucose, dextrose, or the like. The compositions can contain auxiliary substances such as wetting, dispersing, or emulsifying agents (e.g., methylcellulose), pH buffering agents, gelling or viscosity enhancing additives, preservatives, flavoring agents, and/or colors, depending upon the route of administration and the preparation desired. Standard texts may in some cases be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, and sorbic acid. Prolonged absorption of the injectable pharmaceutical form can be brought about by the use of agents delaying absorption, for example, aluminum monostearate and gelatin.

The formulations to be used for *in vivo* administration are generally sterile. Sterility may be readily accomplished, e.g., by filtration through sterile filtration membranes.

Formulations of cells of the disclosure may be free, or substantially free, from cells which are: CD90$^+$; CD105$^+$; CD45$^-$; and have high TIMP-1 secretion and high MMP13 gene expression.

## Doses and therapeutically effective amounts of cells

The therapeutically effective amount of cells of the disclosure may be selected with reference to the nature of the disorder to be treated, and also with reference to the severity of the disease in an individual requiring treatment. Considerations such as the age and weight of the recipient may also influence the selection of an appropriate dose.

A therapeutically effective amount of cells of the disclosure (such as in the form of a medicament disclosed herein) may be provided in a single incidence of treatment, or by multiple incidences of treatment.

A suitable number of therapeutically effective MSC-derived cells for use as disclosed herein may be calculated on the basis of the number of cells administered per site of injury.

Merely by way of example, for treatment of osteoarthritis or other damage to the knee, a dose of between 10 and 20 million cells per knee may prove therapeutically effective. This guidance may be adjusted depending on the organ to be treated, or for larger or smaller sites of injury.

The disclosure will now be further described with reference to the following Examples.

## EXAMPLES

Bone marrow-derived mesenchymal stromal cells (MSCs) are defined as stem cells in part because of their capacity for multipotent differentiation, however more recently they have been shown also to have trophic and immunoregulatory properties that may be important for promoting tissue repair. To assess the hierarchical importance of multipotent differentiation compared with trophic/immunoregulatory functions of MSCs, we have undertaken a detailed analysis of how these properties change with *in vitro* ageing of the cells. Using *in vitro* differentiation and repair models and integrating transcriptomics and proteomics data using Ingenuity Pathway Analysis, we show that the multipotent differentiation capacity of MSCs falls with increasing passage, whereas trophic repair and immunoregulatory potency are maintained even after extensive *in vitro* ageing of the cells. These observations support the view that trophic repair and immunoregulation are core properties of MSCs and therefore hierarchically more important than the transient multipotent differentiation.

## *INTRODUCTION*

The concept of multipotent mesenchymal stem cells (MSCs) was established by Caplan in the early 1990s (Caplan, 1991) following the seminal work of Friedenstein in the 1960s and 1970s, demonstrating the presence of osteogenic precursor cells in bone marrow (Friedenstein et al., 1970; Friedenstein et al., 1968; Friedenstein et al., 1966).

Building on this early work, there have been many studies demonstrating the capacity of MSCs to differentiate *in vitro* with clear evidence for multipotent skeletal lineage differentiation (Kolf et al., 2007), including osteogenesis (Gronthos et al., 1994; Pound et al., 2006; Pound et al., 2007; Simonsen et al., 2002), chondrogenesis (Dickinson et al., 2017; Johnstone et al., 1998; Kafienah et al., 2002; Kafienah et al., 2007b; Kafienah et al., 2006; Martin et al., 1997; Solchaga et al., 2005; Yoo et al., 1998) and adipogenesis (Dickinson et al., 2017; Mirmalek-Sani et al., 2006; Munir et al., 2017) as well as more limited evidence for pluripotent differentiation including endodermal and ectodermal pathways (Caplan, 1991; Kuroda et al., 2011). Some of these studies have demonstrated that clonal populations of bone marrow MSCs retain the capacity for multipotent differentiation (Dickinson et al., 2017; Muraglia et al., 2000), suggesting that at least some individual MSCs within the heterogeneous marrow-derived population exhibit stem cell properties.

More recently, evidence has grown that MSCs may support tissue repair through mechanisms that do not directly relate to their multipotential differentiation capacity (Prockop, 2007). Caplan has described MSCs as having "trophic" capacity by which, following implantation, they induce neighbouring cells to secrete active molecules, for example in the treatment of stroke, myocardial infarction or in meniscal cartilage repair (Caplan and Dennis, 2006). Trophic repair is most likely mediated through the production by MSCs of large amounts of growth factors and other mediators (Caplan and Correa, 2011; Caplan and Dennis, 2006; Kuroda et al., 2011; Prockop, 2009; Tolar et al., 2010). We have previously developed a therapeutic strategy for meniscal cartilage repair based on the trophic properties of MSCs (Pabbruwe et al., 2010b) that has shown some evidence of efficacy in pre-clinical and clinical trials (Whitehouse et al., 2017). A second mechanism of tissue repair that is independent of multipotent differentiation is the ability of MSCs to suppress immune responses by a range of mechanisms including downregulation of T cell proliferation (Dickinson et al., 2017; Keating, 2012; Spaggiari et al., 2007; Tolar et al., 2010; Uccelli et al., 2006; Uccelli et al., 2007). This important property of MSCs has been used clinically to support the engraftment of donated haematopoetic cells and to prevent graft versus host disease (Lazarus et al., 2005; Tolar et al., 2010).

The growing complexity in our understanding of the mechanisms of action of MSCs combined with recent concerns over the lack of rigorous evidence relating to their physiological role has led some to question whether they should be considered as stem cells (Bianco et al., 2008; Caplan, 2017; Kuroda et al., 2011; Sipp et al., 2018). Yet despite these uncertainties, it remains the case that MSCs are being used for a wide range of clinical investigations for tissue regeneration (e.g. bone and cartilage; cardiovascular disease) as well as for disease modification, including haematological disease, graft-versus-host disease and inflammatory diseases (Squillaro et al., 2016). Some of these clinical approaches are based on differentiation whilst others depend on trophic or immunoregulatory functions (Caplan and Correa, 2011), yet we understand little about how these different mechanisms of action relate to each other.

There have been several studies describing the loss of differentiation capacity with increasing passage of MSCs *in vitro* (Bonab et al., 2006; Muraglia et al., 2000; Yang, 2018; Yang et al., 2018), with other studies suggesting that *in vivo* ageing also leads to a loss of differentiation capacity after *ex vivo* isolation of the aged cells (Choudhery et al., 2014; Ganguly et al., 2017; Stenderup et al., 2003). These observations combined with parallel observations of shortening in telomere length with *in vitro* ageing of MSCs (Baxter et al., 2004) have been interpreted as indicating a rapid senescence process that compromises cell function and limits their clinical utility. Conversely, no comparative ageing-related data have been reported for trophic repair or immunoregulation. It is therefore currently unclear if the decline in differentiation capacity with ageing of the cells reflects a general decline in the functional capacity of MSCs as they approach senescence or if there is a selective loss of multipotential differentiation.

We propose here that ageing of MSCs *in vitro* will provide a framework in which to understand the relationship between different aspects of their biology, by identifying those functional properties that are transient and those which are core properties that are retained throughout the lifespan of the cells. The aim of this study was therefore to determine the relative hierarchical importance of differentiation potential and trophic repair/immunoregulation by determining the rate at which these functions decline with increasing MSC ageing *in vitro*.

*RESULTS*

*Patient characteristics and MSC growth potential*

Bone marrow was collected from patients undergoing arthroplastic surgery for treatment of traumatic knee injury. All patients gave informed consent and the study was performed in full accordance with local ethics guidelines (Southmead Research Ethics Committee Ref 078/01). Table S1 shows the patient characteristics. All four were male with a mean age of 49 years (range, 38-70 years) at the time of operation. MSCs were isolated from each bone marrow by plastic adhesion and grown under standard culture conditions until they failed to proliferate any further. MSCs from PN241 and PN242 continued to proliferate for a larger number of passages than MSCs from PN251 and PN264, with PN242 cells showing no sign of growth arrest even at passage 30 (Figure 1A and Table S1). Figure **1B** shows the MSC population doubling time (PDT) at each passage for each patient up to passage 17. The PDT of MSCs from all four patients was similar at early passages and became longer at later passages, with MSCs from one patient (PN264) showing particularly slow growth at higher passage numbers. Early passage MSCs had a typical stellate appearance that was lost as cells became more senescent **(Figure 1C, PN251).** However, PN242 cells had a spindly, stellate appearance even at very high passage number, when they were continuing to proliferate well **(Figure 1C**, **PN242).**

Cell surface marker expression was determined by FACS using MSCs from all four patients at each of passages P1, P5, P10 and P15. The expression of MSC markers CD105 and CD90 was maintained at >90% at late passage in MSCs from all four patients. Haematopoetic stem cell marker CD45 remained at <10% at all passages however there was a small increase in the expression of haematopoetic stem cell marker CD34 with increasing passage number in two of the patients **(Figure 1D**).

***The tri-lineage differentiation potential of MSCs falls with increasing passage***

MSCs from selected passages of each patient were tested for their chondrogenic potential in a three-dimensional cartilage tissue engineering assay, with the amount of engineered cartilage measured as the dry weight of tissue and quality of the cartilage measured as the glycosaminoglycans content expressed as a % of dry weight. There was clear evidence of a loss of chondrogenic potential with *in vitro* ageing of the cells. The typical macroscopic appearance of tissue engineered cartilage over a range of passages for MSCs from each of the patients can be seen in **Figure 2,** clearly demonstrating a reduction in the average size of cartilage constructs when produced using late passage MSCs. This macroscopic observation was supported by quantitative analysis. There was a significant negative correlation between the mean dry weight of cartilage formed and the cumulative population doublings of MSCs recorded at the time of tissue engineering for three out of the four patients and a significant negative correlation between the mean cartilage glycosaminoglycans content and the cumulative population doublings of MSCs recorded at the time of tissue engineering for all four patients **(Figures 3A-D).** Furthermore, both the dry weight and the glycosaminoglycans content of all the individual tissue engineered cartilage constructs were significantly negatively correlated with the passage number of the MSCs used for tissue engineering **(Figures 3E and 3F)**

MSCs were also tested for their osteogenic potential in monolayer culture by semi-quantitative analysis of the staining for alizarin red. Typical images of the staining patterns and the scoring systems used are shown in **Figures S1.** There was some evidence of a gradual loss of osteogenic potency of MSCs however the results were inconsistent between patients. For Patient PN241 there was no apparent change in the mean osteogenic score with cumulative population doublings **(Figure S2A).** For Patient PN242 there was a significant fall in osteogenic potential with cumulative population doublings **(Figures S2B).** PN251 osteogenic scores had declined to 0 by ten population doublings and remained at 0 for higher doublings, although the change was not statistically significant **(Figure S2C).** Patient PN264 exhibited no observable osteogenic activity even at the earliest number of population doublings **(Figure S2D).** Overall, the individual osteogenic scores for the repeated assays were not significantly correlated with the passage number of the MSCs used **(Figure S2E),** however the different patterns observed with each of the patients are indicative of a gradual, if variable decline in osteogenesis with *in vitro* ageing of the cells.

The adipogenic potential of MSCs was tested in monolayer culture by semi-quantitative analysis of the staining for Oil-Red-O. Typical images of the staining patterns and the scoring systems used are shown in **Figures S3.** For Patient PN251 there was a small but significant fall in adipogenic potential with cumulative population doublings **(Figure S4C).** whereas there was no significant change for the other three patients **(Figures S4A, S4B and S4C).** Overall, the individual adipogenic scores for the repeated assays were not significantly correlated with the passage number of the MSCs used **(Figure S4E)** and therefore only very limited evidence for a fall in adipogenesis with *in vitro* ageing of the cells.

Collectively, these data indicate a clear loss of differentiation potential of MSCs, with early passage cells tending to show full tri-lineage potential and later passage cells tending to retain only adipogenic potential or adipogenic plus osteogenic but not chondrogenic potential. These data suggest that multipotential differentiation is not a fundamental property of CD105+ve, CD90+ve, CD34-ve, CD45-ve MSCs, but rather a feature of freshly isolated MSCs that is lost with cell expansion *in vitro*.

***Trophic repair and immunoregulation by MSCs are maintained with increasing passage***

We used the *in vitro* integration of two pieces of meniscal cartilage by MSCs as a model of trophic repair based on our previous studies of this system *in vitro,* in an *in vivo* sheep model and in patients with torn meniscus (Whitehouse et al., 2017). MSCs from all four patients at each of 17 passages were tested for their potency in our meniscal repair potency assay. The variation in % integration of meniscus in these studies was in the same range as previously described, as illustrated in typical histological images **(Figures 4A-C).** In contrast to our observations of tri-potential differentiation (see above), there was no significant change in the mean potency of meniscal repair with cumulative population doublings for any of the four patients**(Figures 4D-G**). Furthermore, the % integration measured for the individual meniscal constructs showed no correlation with the passage number of the MSCs used **(Figure 4H)**

As well as stimulating tissue repair responses, MSCs are also able to suppress immunity through inhibition of T-Cell proliferation and other mechanisms. We therefore measured the % inhibition of T-cell proliferation by MSCs from selected passages for all four patients. As with meniscal repair, there was no significant change in the immunoregulatory capacity of MSCs with increasing passage **(Figure 4I).**

Taken together our observations of trophic meniscal repair and inhibition of T-cell proliferation indicate that the protective and reparative effects of MSCs are a fundamental feature of CD105+ve, CD90+ve, CD34-ve, CD45-ve MSCs, that is retained even after extensive expansion *in vitro.*

*Transcriptomics and proteomics data show significant differences over passage that can be linked to loss of multipotency*

We reserved mRNA from the undifferentiated MSCs of all four patients at each passage. Based on growth and differentiation characteristics we selected mRNA from passages P1, P5, P10 and P15 for gene array comparison. For each of these passages we also collected conditioned medium for proteomic comparison. The genomic and proteomic data were combined and analysed for patterns of change in related genes and proteins. The methodological approach to transcriptomic and proteomic analysis is illustrated in Figure 5A. Data structure was appraised via principal component analysis (PCA). A score plot of the first two principal components is shown in Figure 5B. The changes captured over passage are accounted for in PC2 whilst PC1 captures considerable variation between patients which can be stratified in two groups (shown with blue and orange ellipses). These two groups of patients also show differences in osteogenic capabilities (see above). With the aim of identifying genes and proteins changing solely over passage, independently of this patient variability, we performed a 2-way ANOVA for each variable, as described in the methods section. **Figure 5C** shows the significant variables of these tests. Only 338 genes and proteins are significant uniquely over passage, independently of patient to patient variation or any related iteration. These 338 genes and proteins were used to calculate PCA, of which the first two principal components are shown in **Figure 5D** together with a heatmap of the 338 variables selected. As expected, these genes and proteins show a very clear separation of all the patient samples by passage with a subset decreasing their expression/abundance over passage (top half of the heatmap) while others increase their expression/abundance (bottom half of the heatmap).

For each of these 338 significant proteins and genes we calculated the fold change with respect to passage 1 and analysed these data with Ingenuity Pathway Analysis (IPA) (QIAGEN Inc, https://www.qiagenbioinformatics.com/pro ducts/ingenuity-pathway-analysis). IPA's core analysis, overlaid with the global molecular network within the software resulted in the identification of a number of canonical pathways, functions and upstream regulators found to be significantly over-represented within this list and therefore linked to the loss of multipotenial differentiation capacity of the cells. The largest and most significant change was in the cell-cycle master regulator FOXM1 gene pathway, with clear evidence from transcriptomic data for downregulation of the FOXM1 gene itself (Figure 6A) and with six out of seven of its downstream effectors also predicted by IPA to be downregulated (Figure 6B). Other upstream regulators predicted to be deactivated were the prostaglandin receptor PTGER2, and members of the VEGF family, whilst upstream regulators that were also predicted to be positively activated over passage included the proliferation regulator NUPR and the cytoskeleton regulator MYOC. Quantitative data from our transcriptomic analyses and the associated IPA predictions of changes in the downstream effectors of these regulators, are shown in **Figure S5,** however none of the changes and predicted downstream effects were as clear-cut as for FOXM1.

*Regulators of cell migration and wound healing can be linked to the trophic properties of MSCs*

As shown in Figure 4, the trophic repair capacity of MSCs remains unchanged with increasing passage. With the aim of further investigating this phenomenon we hypothesised that (a) the proteins and genes involved in regulating this functions should not present a significant change in abundance/expression over passage and (b) functions related to cell movement, cell migration and wound healing are likely to be mechanistically involved in trophic repair. To investigate this hypothesis, we used the IPA database to identify the proteins and genes involved within the three terms listed above. Then we mapped those lists to our data, extracted overlapping variables and appraised their significance over passage. The large majority of the proteins and genes mapping to these search-terms were found to be unchanged over passage and expressed at a consistent level across all four passages of our MSC cultures **(Figure 6B),** supporting the hypothesis that genes and proteins expected to be necessary for trophic repair continue to be expressed in ageing cells, when multi-potent differentiation capacity has been lost but trophic repair capacity remains high. We considered CXCL12 (also called Stromal cell-derived factor 1) to be of particular importance at it is associated with all three of our search terms (Figure **6C**) and was expressed consistently highly at both gene and protein level **(Figures 6D and E).**

*Marker genes and proteins*

The IPA analysis outlined above demonstrated downregulation of the FOXM1 canonical pathway with increasing passage/loss of multi-potent differentiation and continuous expression of CXCL12 and other cell migration and wound healing genes and proteins with increasing passage. However, we consider it necessary also to identify genes and proteins that may not be part of canonical pathways or gene/protein families, but that can be used as specific markers of cellular ageing *in vitro.* Such markers would aid in comparison of studies of cells from one laboratory to another or in determining the functionality of an MSC population being used for therapeutic purposes. We therefore analysed the gene array and protein data to identify candidate markers.

Within the significant variable genes identified by transcriptomic analysis, there was a significant decrease with increasing passage in the gene for matrix metalloproteinase 13 (MMP13; Collagenase 3; **Figure 7A** and **Table S2)** and a significant increase with increasing passage in the gene for Insulin-like growth factor binding protein 5 (IGF Binding protein 5; **Table S3).** There was no significant change in other MMP genes **(Table S2)** or IGFBP genes **(Table S3),** nor in any of the genes of the Transforming Growth Factor family **(Table S4).** Because the transcriptomic data indicated that the rate of fall in MMP13 gene expression with passage mirrored closely the fall in chondrogenic potential (compare **Figure 7A** with **Figure 3),** we went on to validate these results using quantitative PCR to determine more accurately the changes in MMP13 gene expression with increasing passage. The results confirm a continuous decline in MMP13 gene expression with increasing passage **(Figure 7B),** demonstrating that loss of MMP13 gene expression could be used to help determine the extent to which MSCs have aged through *in vitro* proliferation.

Within the proteomic dataset we identified those proteins expressed at highest abundance at all passages **(Table S5).** The most abundant of these proteins was Metalloproteinase Inhibitor 1 (Tissue Inhibitor of Metalloproteinase 1; TIMP1; **Table S5 and Figure 7C).** We went on to validate these results using an ELISA kit assay to determine more accurately the changes in TIMP-1 protein secretion with increasing passage **(Figure 7D),** demonstrating that expression of TIMP-1 is a defining characteristic of MSCs, irrespective of the extent to which they have aged through *in vitro* proliferation. Furthermore, when MSCs were seeded onto collagen scaffolds and cultured for 24h, the cells continued to secrete high levels of MSCs from the cell/scaffold constructs into their culture medium, however if the constructs were freeze-thawed under conditions that reduced their viability, the secreted TIMP levels were substantially reduced **(Figure 7E).**

We conclude that MSCs expressing high levels of the MMP13 gene whilst secreting abundant TIMP-1 could potentially be used for therapeutic processes involving either phenotypic differentiation or trophic/immunoregulatory repair. However, MSCs expressing low levels of the MMP-13 gene whilst secreting abundant TIMP-1 could only be used for processes involving trophic/immunoregulatory repair, but they could not be used reliably for those processes involving differentiation.

## *DISCUSSION*

We have shown that the loss of multipotent differentiation capacity of MSCs with extensive passaging *in vitro* cannot be the result of a generalised loss of cell function as the MSCs senesce, since two other important properties of MSCs (trophic repair and immunoregulation) are not significantly reduced even after a very high number of population doublings and reduced differentiation capacity was observed many passages before cessation of proliferation. Furthermore, families of genes and proteins from a range of critical signalling pathways show clear changes that correlate with increasing passage number and loss of differentiation, whereas genes and proteins known to be involved with wound repair and cell movement/migration do not vary significantly with increasing passage number. Downregulation of the Forkhead Box M1 (FOXM1) canonical pathway showed a clear relationship to passage number, indicating a potential role for FOXM1 in the maintenance and then loss of multipotency. This observation is consistent with previous studies of its biological function. It is a proto-oncogene that is a key master regulator in the survival of cancer stem cells (Nakano, 2014; Xie et al., 2010). It has also been shown to be highly expressed in multipotent and pluripotent stem cells and to be critical to the maintenance of stem cell potency (Besharat et al., 2018; Youn et al., 2017) and to the induction of pluripotency through reprogramming (Jeong et al., 2017). CXCL12 (also known as SDF-1) was found to be associated with all three of our search terms related to trophic repair and its gene and protein levels were maintained even at very late passage numbers, indicating a potential role for CXCL12 in MSC-mediated trophic repair. This observation is consistent with previous studies which have demonstrated its critical role in MSC-mediated induction of spinal cord repair (Stewart et al., 2017) and myocardial repair (Dong et al., 2012), as well as enhancing nerve cell survival in vitro (Chalasani et al., 2003) and mediating trophism between endothelial cells and tumour cells (Rao et al., 2012). In addition to these mechanistic relationships, we identified two functional markers relating to MSC ageing in vitro, namely the MMP13 (Collagenase 3) gene, which is downregulated with increasing passage, and TIMP-1 protein, which is the most abundant protein in the secretome at all passages. It is particularly interesting that these two markers, which were identified independently of each other (one from transcriptomics data and one form proteomics), are biologically related to each other as TIMP-1 is an inhibitor of MMP13 and as well as other metalloproteinases.

Previous studies have clearly shown that multipotency of MSCs declines with both *in vitro* and *in vivo* ageing (Bonab et al., 2006; Choudhery et al., 2014; Ganguly et al., 2017; Muraglia et al., 2000; Stenderup et al., 2003; Yang, 2018;

Yang et al., 2018). Our observation here that chondrogenesis declines, osteogenesis tends to decline, but adipogenesis is retained at higher passage number is in agreement with the work of Yang et al (Yang et al., 2018). Muraglia et al (Muraglia et al., 2000) also showed a loss of multipotent differentiation with passage, but in their experiments, osteogenesis was retained with early loss of adipogenesis. However, they were working with clonal MSC cell-lines whereas the work reported here and by Yang et al investigated the whole MSC population.

Haynesworth et al first described the unique cytokine expression pattern of MSCs (Haynesworth et al., 1996) and they demonstrated a reduction in cytokine production after stimulating differentiation using dexamethasone. A decade later, Caplan and Dennis coined the term "trophic effect" which they defined as "those chemotactic, mitotic, and differentiation-modulating effects which emanate from cells as bioactive factors that exert their effects primarily on neighbouring cells and whose effects never result in differentiation of the producer cell" (Caplan and Dennis, 2006). They cited, as a typical example of this effect, the support provided by MSCs in the bone marrow niche for growth and differentiation of haematopoetic cells. They also summarised evidence for MSCs providing trophic support in a range of cell therapy settings including the treatment of stroke, myocardial infarction and meniscal cartilage regeneration. Numerous other studies have gone on to describe the importance of MSC-induced trophic repair (Caplan and Correa, 2011; Caplan and Dennis, 2006; Kuroda et al., 2011; Prockop, 2009; Tolar et al., 2010). We exploited the trophic effects of MSCs in devising a new method of treating fresh meniscal cartilage tears using a stem cell/collagen-scaffold implant to promote integration of the damaged tissue. Our original *in vitro* studies demonstrated that the method depends on cell migration from the implant into surrounding tissue and interaction with the endogenous meniscal cells (Pabbruwe et al., 2009; Pabbruwe et al., 2010b). Importantly, we found that MSCs that have been stimulated with Transforming Growth Factor ß to undergo chondrogenic differentiation are much less potent at promoting meniscal repair than the undifferentiated MSCs (Pabbruwe et al., 2010b). We went on to describe the use of undifferentiated MSCs seeded on a collagen scaffold to repair meniscal injury in a sheep pre-clinical model and in a first in human trial (Whitehouse et al., 2017). In the current study we used our *in vitro* semi-quantitative meniscal cartilage integration assay (Pabbruwe et al., 2010b) as a model for trophic repair and made the very surprising observation that MSCs that have been cultured for up to 30 passages retain the same capacity for trophic repair as very early passage cells. These functional data were supported by our analysis of genes and proteins involved with cell movement and migration and with wound healing, showing that unlike those linked to differentiation, there was no significant change in their expression with increasing passage.

The immunoregulatory effects of MSCs have been widely described and although a range of mechanisms are involved, it is clear that inhibition of T-cell proliferation is a critical component of their suppressive activity (Dickinson et al., 2017; Keating, 2012; Spaggiari et al., 2007; Tolar et al., 2010; Uccelli et al., 2006; Uccelli et al., 2007). Human T-cells can be strongly stimulated to proliferate using a combination of anti-CD3 and anti-CD28 antibodies (Verhagen and Wraith, 2014). Their rate of proliferation can be monitored by covalently labelling intracellular molecules with a fluorescent dye that is then diluted out by 50% with each cell division, tracked by FACS (Quah et al., 2007). Adding MSCs into cultures of labelled, stimulated T-cells suppresses the lymphocyte proliferation, so prolonging the accumulation of the dye (Dickinson et al., 2017). In the current study, we have used this method to measure the immunoregulatory effects of MSCs at early and late passage and found no loss of potency with increasing passage. For human MSCs, the mechanism of T-cell suppression has been described as involving indoleamine 2,3-dioxygenase-mediated tryptophan degradation (Meisel et al., 2004). Taken together, these results demonstrate that, as with trophic repair, immunoregulation is a fundamental property of MSCs that is not lost with increasing passage.

Previous studies have highlighted the loss of multipotent differentiation capacity of MSCs with increasing passage and this deficit has been assumed to be related to progression of the cells to a senescent end-state (Bonab et al., 2006; Muraglia et al., 2000; Yang, 2018; Yang et al., 2018). However, the results described here demonstrate that even after extensive passaging *in vitro,* there is no apparent loss of trophic repair or immunoregulation potency, indicating that in these respects, MSCs remain fully functional even just a few days before they cease to proliferate. We can therefore conclude that there is a hierarchy to the measurable functions of MSCs, with trophic repair and immunoregulation being core properties whereas multipotency is a transient property *in vitro.*

The term "Mesenchymal Stem Cells" was coined by Caplan (Caplan, 1991), who went on to describe MSCs as "An injury Drugstore" (Caplan and Correa, 2011) and more recently advocated a change in their name to "Medicinal Signaling Cells" (Caplan, 2017). Other studies have questioned the definition of MSCs as stem cells because of the lack of rigorous confirmatory biological evidence (Bianco et al., 2008; Javazon et al., 2004; Keating, 2012; Kuroda et al., 2011; Prockop, 2009), with all of these studies calling for more experimental data before reaching a conclusion on the nomenclature. Others have been more forthright in concluding that MSCs are not stem cells and have called for an immediate change in nomenclature, in order to avoid the over-hyped marketing of MSCs as "miracle cures" (Caulfield et al., 2016; Sipp et al., 2017; Sipp et al., 2018). Prockop emphasised that the essence of a stem cell should not be determined by its status at a single point in time (Prockop, 2009). His insightful review provides a useful context for the data reported here. In this study, we have investigated the *in vitro* differentiation and trophic behaviour of MSCs across many passages over time and in this way, have reached the conclusion that the property of multipotency is relatively transient whereas the trophic effects of these cells is apparently permanent all the way through to the time of growth arrest.

In conclusion, our findings emphasise the hierarchical importance of trophic repair over multipotency and provide some additional evidence in favour of MSCs being defined as repair cells rather than mesenchymal stem cells.

## STAR METHODS

## CONTACT FOR REAGENT AND RESOURCE SHARING

Further requests for reagents may be directed to, and will be fulfilled by, the Lead Contact, Anthony Hollander (A.Hollander@Liverpool.ac.uk).

## EXPERIMENTAL MODEL AND SUBJECT DETAILS

### Isolation and expansion of human marrow derived MSCs for *in vitro* studies

The model used as the basis for all the experiments reported here was long-term culture of human bone-marrow derived MSCs. Bone marrow plugs were collected from the femoral heads of patients undergoing total hip replacement. All patients gave their informed consent and the study was carried out according to local ethical guidelines (North Bristol NHS Trust Research Ethics Committee). Patient details can be seen in Table S1. Cells were suspended in stem cell expansion medium consisting of low glucose Dulbecco's Modified Eagles Medium (Sigma) supplemented with 10% (v/v) Foetal Bovine Serum (FBS, Thermo Scientific Hyclone, Loughborough, UK), 1% (v/v) Glutamax (Sigma) and 1% (v/v) Penicillin/Streptomycin (Sigma). The serum batch was selected to promote the growth and differentiation of MSCs (Kafienah et al., 2007a). The medium was also supplemented with 10 ng/ml FGF-2 (Peprotech). This growth factor has been previously shown to enhance the MSC proliferation rate *in vitro* (Bianchi et al., 2003; Solchaga et al., 2005), to retain MSCs as undifferentiated cells during proliferation (Kafienah et al., 2006; Martin et al., 1999) and to enhance chondrogenic differentiation when the FGF-2 expanded MSCs are subsequently exposed to differentiation conditions (Bianchi et al., 2003; Solchaga et al., 2005). The cell suspension was separated from any bone in the sample by repeated washing with media. The cells were centrifuged at 500 $g$ for 5 minutes and the supernatant/fat removed. The resulting cell pellet was resuspended in medium, and then plated at a seeding density of between 1.5-2.0x$10^5$ nucleated cells per cm$^2$. These flasks were incubated at 37°C in a humidified atmosphere of 5% $CO_2$ and 95% air. Four days were allowed before the first medium change and then the medium was changed every other day until adherent cells reached 90% confluence and were ready for passaging.

## METHOD DETAILS

### Cell passaging and calculation of population doublings and doubling time

At the end of each passage the MSCs were harvested using 0.25% trypsin-EDTA (Invitrogen), pooled, counted and then divided into different centrifuge tubes for reseeding and further growth, for immediate use in measurement of % integration of meniscal cartilage, for storage in liquid nitrogen for subsequent use in differentiation protocols as well as for genomic and proteomic analysis. The cells for each patient were passaged continuously without freezing, until growth arrest, defined as no detectable increase in cell number between passages (See Table S1). At each passage, the total number of harvested MSCs was determined. The first cell harvest after seeding of fresh bone marrow was taken as passage 0. The number of cells reseeded at the start of Passage 1 was used as the baseline for calculation of the first population doubling value at the end of passage 1. Downstream analyses of the MSCs were undertaken from passage 1 onwards.

The number of Population doublings (PDs) was calculated using the following formula:

$$PDs = [log(\text{Number of Harvested MSCs}) - log(\text{Number of seeded MSCs})]/log(2)]$$

The PD for each passage was calculated and added to the PD of the previous passages to generate data for Cumulative PD at each Passage.

Population doubling time (PDT) was calculated for each passage using the formula:

$$PDT = t \times log(2)/log(\text{cells harvested/cells seeded})$$

(t = the time between cell seeding and cell harvesting)

**Detection of cell-surface phenotypic markers**

MSCs (100,000 cells from each patient at each of passages 1, 5, 10 and 15) were suspended in a 1:500 dilution of Zombie (Biolegend), a live/dead cell dye, and incubated for 20mins in the dark. Nonspecific antigens were then blocked by incubating the cells at room temperature for 1 hour in 1% (wt/vol) BSA (Sigma-Aldrich), 5% (vol/vol) FCS (Sigma-Aldrich), and 10% (vol/vol) human serum (Sigma-Aldrich). The cells were washed by centrifugation in three volumes of PBS, and the cell pellet was suspended in 100µl of a primary antibody solution containing 20-100µg/ml of antibody in blocking solution. All the primary antibodies were fluorescent-labelled mouse anti-human IgGs: anti-CD105-fluorescein isothiocyanate (FITC), anti-CD90-phycoerythrin (PE), anti-CD45-PE were from R&D Systems); anti-CD34-FITC was from BD Bioscience; IgG1-FITC and IgG1-PE isotype controls were from R&D Systems. After incubation for 40 minutes at 4°C, the cells were washed and suspended in 1 ml of PBS for analysis on a Canto flow cytometer (BD FACSCanto II), after the exclusion of non-viable cells. Data were analysed using FlowJo (Treestar). Positive expression was defined as the level of fluorescence greater than 95% of corresponding isotype-matched control antibodies.

**Chondrogenesis**

*Cartilage tissue engineering*

The chondrogenic capacity of MSCs from each passage were assessed by performing three-dimensional cartilage tissue engineering, as previously described (Kafienah et al., 2007a). Briefly, 300,000 cells were loaded drop-wise onto 5 mm diameter x 2 mm thick polyglycolic acid (PGA) scaffold discs (Biomedical Structures, Warwick, RI, USA) which had been precoated with 100 µg/ml fibronectin (Sigma). Constructs were then cultured in chondrogenic differentiation medium consisting of DMEM containing 4500 mg/L glucose (Sigma-Aldrich), supplemented with 10 ng/ml transforming growth factor-β3 (TGF-β3; R&D Systems), 100 nM dexamethasone, 80 µM ascorbic acid 2-phosphate, 1 mM sodium pyruvate, 1% (v/v) Penicillin/Streptomycin (all from Sigma-Aldrich), 1% insulin-transferrin-selenium-G (ITS) and 2 mM Glutamax-I (both from Invitrogen). After 7 days, the medium was further supplemented with 10 µg/ml bovine pancreatic insulin (Sigma-Aldrich) until the end of culture. The constructs were incubated at 37°C for a total of 35 days on a rotating platform and medium was changed every three days.

*Biochemical analysis*

Cartilage constructs were freeze-dried and weighed at the end of the 35-day tissue engineering period. The extracellular matrix was fully solubilised by overnight digestion with 2 mg/ml bovine pancreatic trypsin (Sigma-Aldrich) which was then boiled for 15 min to inhibit the action of the enzyme (Dickinson et al., 2005). In order to obtain the dry weight of extracellular matrix in the construct, remaining undigested scaffold material was freeze-dried, weighed and subtracted from the original dry weight. The amounts of proteoglycan in the digests was measured as sulphated glycosaminoglycan (GAG) using a dimethylmethylene blue (Sigma-Aldrich) colorimetric assay (Handley and Buttle, 1995).

**Osteogenesis and adipogenesis**

Whole MSC populations or MSC clones were grown in monolayer until 50-70% confluent prior to osteogenic differentiation or 100% confluent prior to adipogenic differentiation. In both cases, control cells were then cultured in α-MEM (Invitrogen) basal medium containing 10% FBS, 1% (v/v) Penicillin/Streptomycin and 2 mM Glutamax-I. Cells stimulated to undergo differentiation were cultured in basal medium containing either Osteogenic Supplement or Adipogenic Supplement (both from R&D Systems) for 21 days. Following osteogenic differentiation cells were fixed in 70% ethanol and stained with 40 mM alizarin red S (Sigma-Aldrich), pH4.1, for 5 min. Following adipogenic differentiation cells were fixed in 4% paraformaldehyde and stained with 0.3% oil red O (Sigma-Aldrich) for 30 min. The extent of differentiation was scored under blind conditions and classified as - (no staining), +, ++ or +++ according to increasing area and number of mineralised deposits following osteogenic differentiation (See **Figure S1)** and increasing number of lipid droplets following adipogenic differentiation (see **Figure S3).**

**Meniscal repair**

*Preparation of sheep meniscal cartilage*

Sheep legs were purchased from Edge & Son Butchers, Wirral, Merseyside and the meniscal cartilage removed under sterile conditions. Meniscal cartilage cylinders (5.0 mm in diameter and 3.0 mm thick) were harvested from the avascular (white zone) of the ovine menisci using a dermal biopsy punch. They were rinsed and incubated with phosphate buffered saline (PBS; Invitrogen Ltd, Paisley, UK) containing 10% (v/v) Penicillin/Streptomycin (Sigma-Aldrich) and 1% (v/v) 250$\mu$g/ml Amphotericin B (Sigma-Aldrich) for 20 minutes. Viability of the fibrocartilage discs was maintained by culture in basic medium consisting of low-glucose DMEM with 10mM Hepes buffer (Sigma), 1% (v/v) Penicillin/Streptomycin, non-essential amino acids (NEAA; Sigma), 1% (v/v) Glutamax and 10% amphotericin B at 37°C in a 5% $CO_2$ environment. The explants were used in the integration experiments within 3 days of culture.

*Cell seeding*

Collagen scaffolds (Ultrafoam Collagen Sponge; Bard, UK, www.barduk.com) were cut into 6mm diameter discs and seeded with human MSCs at a concentration of 1 x $10^6$ cells/cm$^2$. The suspension was loaded drop wise onto the scaffold placed in ultra-low attachment wells of a 24-well plate (Corning®, Acton, USA). After 4 hours, 1.5 ml of expansion medium containing 10 ng/ml FGF-2 was added and changed daily. Seeded scaffolds were incubated for 48 hours at 37°C in an orbital shaker at 50 rpm.

*Assembling and culture of constructs*

Sandwich constructs of two ovine meniscal cartilage discs interposed with a seeded scaffold were assembled as previously described (Whitehouse et al., 2017) using skin clips and cultured *in vitro* in ultra-low attachment 6-well plates in expansion medium with 10 ng/mL FGF-2 for 7 days followed by culture in an integration medium consisting of high glucose DMEM containing 10% (v/v) FBS, 1% (v/v), Glutamax, 1% (v/v) Penicillin/Streptomycin, insulin and ascorbate-6-phosphate (50$\mu$g/ml; Sigma) for 33 days. The medium was replenished twice every week. The constructs were incubated at 37°C on a rotating platform throughout the culture period. At the end of culture, the constructs were prepared for histological analysis by fixation in 10% (v/v) neutral buffered formalin.

*Histomorphometric analysis*

Histomorphometry was carried out using a method that we developed and characterized in previous studies (Pabbruwe et al., 2009; Pabbruwe et al., 2010a; Whitehouse et al., 2017). Fixed constructs were dehydrated and paraffin embedded. Samples were cut into 4$\mu$m sections and stained with hematoxylin and eosin (H&E) for the study of morphological details. All histological sections were scanned using a Leica Aperio slide scanner and histomorphometric analysis was performed under blind conditions, using ImageScope software (Leica). Two perpendicular sections, one at the edge and another one at the center of each construct were used. For each section, the entire length of the implant/meniscus interface was measured, as well as the length of any areas of integration at the interface. The repair index was then determined as:

$$\% \text{ Integration = Length of integrated interface/Total interface length x 100}$$

**Immunosuppression**

Human peripheral blood mononuclear cells (PBMCs) were isolated from donor blood samples from healthy volunteers. All the volunteers gave their informed consent and the study was carried out according to local ethical guidelines (University of Liverpool Research Ethics subcommittee for Physical Interventions). PBMCs were isolated by centrifugation of the blood on 1.077 g/mL Ficoll-Paque (GE Healthcare Life Sciences, Little Chalfont, UK) and cultured in RPMI-1640 containing L-glutamine and supplemented with 10% Human AB Serum and 1% (v/v) Penicillin/Streptomycin. PBMCs were stained with CellTrace™ Violet (ThermoFisher Scientific) to monitor T-cell proliferation. Labelled PBMCs were then stimulated with 3.75$\mu$g/ml anti-human CD3 (HIT3a) and 2$\mu$g/ml anti-human CD28 (CD28.2) (both from Fisher Scientific Affymetrix eBioscience, Cheshire, UK) and co-cultured with MSCs from 4 individual donors at passages 1, 5, 10 and 15 for 72 hours. The T-cell proliferation profile for each population was analysed by flow cytometry following exclusion of non-viable cells stained with 7-amino-actinomycin D (7-AAD; BD Biosciences).

**Quantitative PCR**

Real-time quantitative PCR (RT-qPCR) for MMP13 mRNA and for the housekeeping gene Tata Binding Protein (TBP) were performed using the CellsDirect™ One-Step qRT-PCR Kit (ThermoFisher), with which reverse transcription and PCR amplification were performed in the same reaction tube. Primers specific for MMP13 (Hs00942584_m1) and TBP (Hs00427621_m1) were purchased from ThermoFisher TaqMan®. The reaction was started by synthesising cDNA at 50°C for 15min, followed by 2min at 95°C to denature RNA-cDNA hybrids and deactivate reverse transcriptase. The thermal cycling program consisted of 50°C for 15 minutes, 95°C for 2 minutes, and 40 two-step cycles of 95°C for 10s and 60°C for 30s. MMP13 expression relative to TBP was determined at each of 4 time points for each MSC sample and the results normalised to the time (passage 2), which was taken as a Fold-expression of 1.0.

**TIMP-1 ELISA**

TIMP-1 protein was measured in the secretome of MSCs using the Quantikine® ELISA Kit for human TIMP-1 (R&D Systems). MSCs were seeded into 6-well plates at 2.25 million cells/well (3 replicates ) and cultured in 2ml of DMEM for 24 h. The medium was then replaced with 1 ml of phenol-free culture medium for a further 24h, after which the secretome was collected and assayed at appropriate dilution using the ELISA kit.

**Acquisition of genomic and proteomic data**

*Transcriptomics*

Transcriptomics was performed by the Centre for Genomic Research on mRNA extracted from the mRNA of all four patients at P1, P5, P10 and P15. When MSCs were harvested at the end of each passage, $1 \times 10^6$ cells were isolated and resuspended in RNAprotect Cell Reagent (Qiagen). The cells were stored at -80°C until the complete set of samples from all donors and time points had been collected. RNA was then extracted from selected time points using the RNeasy Plus Mini Kit (Qiagen), according to the manufacturer's instructions. The concentration of RNA in the extract was determined using a NanoDrop 2000 spectrophotometer (Thermo). Extracted RNA was stored at -80°C prior to analysis.
Ribosomal RNA depletion was performed using the Ribo-Zero™ H/M/R Kit (Illumina) and RNASeq libraries were then prepared using the NEB Next Ultra Directional RNA Library Prep Kit (Illumina). Paired-end sequencing of the RNASeq libraries was performed by the Illumina HiSeq4000 platform using V4 chemistry.

*Proteomics*

Proteomics was performed on secretome prepared from the MSCs of all four patients at P1, P5, P10 and P15. When MSCs were harvested at the end of each passage, $1 \times 10^6$ cells were isolated and resuspended in 4 mL of serum-free, phenol red-free DMEM (Sigma) supplemented with 4500 mg/L glucose, 1% (v/v) Glutamax (Sigma), 1% (v/v) P/S (Sigma).and 2 mM Glutamax-I for 24 hours at 37°C. The conditioned medium recovered at the end of the incubation was harvested from each flask and stored at -80°C prior to analysis.
Proteomic analyses of the secretome samples was undertaken in the "Centre for Proteome Research" facility at the University of Liverpool. Protein solutions were concentrated by adding consecutive 1 mL aliquots of each sample to 10μL of Strataclean beads (Stratagene®, Hycor Biomedical Ltd., Edinburgh, UK). After each aliquot had been added, the sample was vortexed for 1 minute, centrifuged at 2,000 x g for 2 minutes and the protein-depleted supernatant removed. After the final aliquot had been added, the beads were washed 2x with 1 mL of 25mM ambic prior to digestion. For on-bead digestion, the beads were re-suspended in 80μL of 25 mM ambic and 5μL of 1%(w/v) Rapigest in 25 mM ambic (Waters Limited, Hertfordshire, UK) was added. The samples were heated at 80°C for 10 minutes and then reduced by the addition of 5 μL of dithiothreitol (DTT, 9.2mg/mL in 25mM ambic) and heating at 60°C for 10mins. Following cooling, 5 μL of iodoacetamide (33mg/mL in 25mM ambic) was added and the samples incubated at RT for 30min in the dark. Porcine trypsin (sequencing grade, Promega) (1μg) was added and the sample was incubated at 37°C overnight on a rotary mixer. The digests were acidified by the addition of 1 μL of trifluoracetic acid (TFA) and incubated at 37°C for 45 minutes. Samples were then centrifuged at 17,200 x g for 30mins and supernatants transferred to 0.5 mL low-binding tubes. They were centrifuged for a further 30mins and 10μL transferred to total recovery vials for LC-MS analysis.
Data-dependent LC-MSMS analyses were conducted on a QExactive HF quadrupole-Orbitrap mass spectrometer coupled to a Dionex Ultimate 3000 RSLC nano-liquid chromatograph (Hemel Hempstead, UK). Sample digest (1-2μL) was loaded onto a trapping column (Acclaim PepMap 100 C18, 75μm x 2cm, 3μm packing material, 100Å) using a loading buffer of 0.1%(v/v) TFA, 2%(v/v) acetonitrile in water for 7 minutes at a flow rate of 12μL min$^{-1}$. The trapping column was then set in-line with an analytical column (EASY-Spray PepMap RSLC C18, 75μm x 50cm, 2μm packing material, 100Å) and the peptides eluted using a linear gradient of 96.2% A (0.1 %[v/v] formic acid): 3.8% B (0.1 % [v/v] formic acid in water: acetonitrile [80:20] [v/v]) to 50% A:50% B over 90min at a flow rate of 300nL min$^{-1}$, followed by washing at 1% A : 99% B for 5 minutes and re-equilibration of the column to starting conditions. The column was maintained at 40°C, and the effluent introduced directly into the integrated nano-electrospray ionisation source operating in positive ion mode. The mass spectrometer was operated in DDA mode with survey scans between *m/z* 350-2000 acquired at a mass resolution of 60,000 (FWHM) at *m/z* 200. The maximum injection time was 100ms, and the automatic gain control was set to 3e6. The 16 most intense precursor ions with charges states of between 2+ and 5+ were selected for MS/MS with an isolation window of

2*m/z* units. The maximum injection time was 45ms, and the automatic gain control was set to 1e5. Fragmentation of the peptides was by higher-energy collisional dissociation using a normalised collision energy of 30%. Dynamic exclusion of *m/z* values to prevent repeated fragmentation of the same peptide was used with an exclusion time of 20s. Raw data files from the mass spectrometry were imported into Progenesis QI for Proteomics v.2.0 software (Waters Ltd, Newcastle upon Tyne, UK) for alignment and peak detection. An aggregate file was generated which contained all the peaks from all runs in an experiment so that there were no missing values. The data was filtered and charges +1 and ≥+8 were removed. The msms fragmentation data was searched against the UniProt human reviewed DB using Mascot v. 2.4.1 software (Matrix Science, London, UK). The precursor ion mass tolerance was set to 10ppm and the product ion tolerance to 0.01Da. Oxidation of methionine was selected as a dynamic modification and carbamidomethyl cysteine as a fixed modification. One missed cleavage was permitted. The Mascot search returned 2,594 proteins at 2.17% FDR (psms above homology). A 1%FDR was set and 2,366 proteins were exported as an .xml file (FDR type: distinct psms) and imported into Progenesis and peptides assigned to proteins. Protein quantification was based on averaging the individual abundances for each protein per donor at each passage and comparing proteins that were differentially expressed between the 4 passages.

## QUANTIFICATION AND STATISTICAL ANALYSIS

### Bioinformatics

Data processing, integration and analyses were undertaken by the Computational Biology Facility at the University of Liverpool. RNAseq data was acquired as described above. The raw Fastq files were trimmed for the presence of Illumina adapter sequences using Cutadapt version 1.2.1, option -O 3. Reads were further trimmed using Sickle version 1.200 with a minimum window quality score of 20. Reads shorter than 10 base pairs after trimming were removed. Sequence quality metrics were assessed using FastQC version 0.11.4. No samples were removed. Sequence data were aligned to the human genome version GRCh38 from NCBI using Bowtie2 version 1.1.2 with recommended parameters (Langdon, 2015). Gene level count data were generated from the Bowtie2 alignments using htseq-count version 0.9.0. The R library DESeq2 was used to produced rlog transformed count data. These were filtered to remove genes with less than 1 average count. Statistical analyses were performed in R version 3.4.4 and graphical representations were done using the R package ggplot2.

Normalised proteomics and transcriptomics data were integrated and preliminary exploratory analyses revealed a relevant heterogeneity between patients. In order to discriminate between changes related to patient heterogeneity and changes related to passage, differentially expressed variables over passage were calculated with a 2-way ANOVA to account for patient variability as confounder. This followed multiple testing correction using the Benjamin-Hochberg method. Significant variables (338 of which 38 were proteins and 300 were genes) uniquely over passage were selected for further analysis in the Ingenuity Pathway Database (IPA, Qiagen, Analysis 2015).

Base 2 log fold changes of the 338 significant variables were calculated with respect to passage 1 and used for a IPA (Qiagen), where analysis was performed according to the manual to predict possible upstream regulators involved in the changes observed, possible pathways affected and a number of functions and terms significantly enriched (database access on the 9th of December 2018). IPA was also used to download knowledge terms involved in selected key processes, i.e. (A) Cell migration in mesenchymal stem cells (B) cell movement in mesenchymal stem cells and (C) wound healing. All these variables were mapped to our experimental data. Then we assessed the number of variables changing over passage within these terms and contextualise the biological findings.

Principal Component Analysis was performed in mean centred and scaled data via singular value decomposition using the function prcomp within the statistical software R.

### Statistics

Differentiation variables (TE, GAG, and osteogenesis and adipogenesis scores) were compared over passage by calculating the non-parametric Spearman correlation embedded in the function cor.test within the stats package in R. Osteogenesis and adipogenesis capabilities were measured with semi quantitative data based on image analysis. These were translated into integers 0 to 1 to undertake the calculation.

**Table S1.** *Related to Figure* 1; Patient details and MSC culture. Bone marrow was obtained from each of four donors undergoing surgery following traumatic injury. Growth arrest was recorded as the last Passage at which an increase in cell number was recorded. Cells from PN242 continued to proliferate at P30, after which the experiment was terminated.

| Donor | Age at surgery (Years) | Gender | MSC passage at growth arrest |
|---|---|---|---|
| PN241 | 45 | M | P25 |

(continued)

| Donor | Age at surgery (Years) | Gender | MSC passage at growth arrest |
|---|---|---|---|
| PN242 | 38 | M | >P30 |
| PN251 | 70 | M | P17 |
| PN264 | 43 | M | P16 |

**Table S2.** *Related to Figure 7;* Mean gene expression levels at each passage from transcriptomic data relating to matrix metalloproteinase family genes. ANOVA was used to test significant variation from P1 through to P15. Significant changes highlighted with italic text.

| Gene | p1 | p5 | p10 | p15 | ANOVA P value |
|---|---|---|---|---|---|
| MMP1 | 5.5998732 | 5.79613531 | 4.95625731 | 5.65660047 | 0.99307064 |
| MMP11 | 7.86223562 | 7.68306861 | 7.18984412 | 6.9289258 | 0.37797616 |
| *MMP13* | *10.0768097* | *7.88922323* | *5.6507086* | *5.1051077* | *0.0392376* |
| MMP14 | 14.1920592 | 14.0462124 | 14.0181522 | 13.7617206 | 0.10282711 |
| MMP15 | 6.62219651 | 6.6322322 | 6.09832576 | 5.87278483 | 0.09315796 |
| MMP16 | 9.47449366 | 9.70117959 | 9.13228025 | 9.1221298 | 0.71596372 |
| MMP17 | 6.61458779 | 6.45119928 | 6.42559403 | 6.25754062 | 0.9183799 |
| MMP19 | 7.89561958 | 7.81847612 | 7.78299404 | 7.52317205 | 0.41654145 |
| MMP2 | 13.9744784 | 13.985333 | 13.9381087 | 13.9339775 | 0.99943649 |
| MMP24 | 6.21874088 | 6.44035293 | 6.60509831 | 6.39380761 | 0.58978511 |
| MMP24-AS1 | 7.33276168 | 7.56962075 | 7.54026812 | 7.37038882 | 0.69985261 |
| MMP25 | 1.43714412 | 1.40793236 | 1.79394523 | 1.56026113 | 0.58544464 |
| MMP28 | 4.78786145 | 4.64276263 | 4.87752538 | 4.81640313 | 0.97791831 |
| MMP3 | 3.54205151 | 2.89678063 | 2.90764104 | 3.09622363 | 0.32241168 |

**Table S3.** *Related to Figure 7;* Mean gene expression levels at each passage from transcriptomic data relating to insulin-like growth factor family genes. ANOVA was used to test significant variation from P1 through to P15. Significant changes highlighted with italic text.

| Gene | p1 | p5 | p10 | p15 | ANOVA P value |
|---|---|---|---|---|---|
| FIGF | 1.766388704 | 1.785539917 | 1.561268748 | 1.784696387 | 0.872544153 |
| IGF1 | 8.36150045 | 7.853231912 | 7.9100807 | 6.95961508 | 0.682343942 |
| IGF1R | 9.525628717 | 9.623276784 | 9.835460052 | 9.984616432 | 0.273174169 |
| IGF2 | 1.797574755 | 1.942635003 | 2.060736917 | 1.87051458 | 0.850417613 |
| IGF2-AS | 2.392545163 | 2.82090428 | 2.981375358 | 2.529064565 | 0.580132182 |
| IGF2BP1 | 4.993120303 | 5.23977081 | 6.051077551 | 5.888056299 | 0.604856443 |
| IGF2BP2 | 8.491727488 | 8.711832275 | 8.387332288 | 8.447843126 | 0.15209104 |
| IGF2BP3 | 6.772587378 | 6.564516998 | 6.566581798 | 6.63153097 | 0.996849241 |
| IGF2R | 10.64727025 | 10.93571433 | 10.89836614 | 10.97222435 | 0.344377129 |
| IGFALS | 0.97232465 | 0.955956548 | 1.252926517 | 0.946706521 | 0.26398872 |
| IGFBP1 | 2.818366246 | 2.85717034 | 2.700755194 | 3.569848744 | 0.427416333 |
| IGFBP2 | 9.671513003 | 10.5682349 | 10.87819621 | 11.17326522 | 0.634062901 |
| IGFBP3 | 14.31915607 | 14.59662532 | 14.56390172 | 15.19442393 | 0.67135429 |
| IGFBP4 | 11.28057242 | 11.64997926 | 11.78187461 | 11.53089188 | 0.781250375 |

(continued)

| Gene | p1 | p5 | p10 | p15 | ANOVA P value |
|------|-----|-----|-----|-----|---------------|
| *IGFBP5* | *12.79305878* | *13.51606844* | *14.45217328* | *15.16636268* | *0.037020368* |
| IGFBP6 | 12.68995546 | 12.73214007 | 13.09245732 | 12.99015695 | 0.831659325 |
| IGFBP7 | 11.57745438 | 11.14780103 | 11.04839444 | 11.01817982 | 0.968411897 |
| IGFBP7-AS1 | 2.505413873 | 2.487770296 | 2.564428641 | 2.463306097 | 0.987864613 |
| IGFBPL1 | 1.560575075 | 1.821169981 | 1.712299146 | 1.550038652 | 0.6999863 |
| IGFLR1 | 4.811177986 | 4.574514281 | 4.343826906 | 4.561327033 | 0.841321237 |
| PIGF | 6.418983207 | 6.635966057 | 6.37149974 | 6.611985474 | 0.467353007 |

Table S4. *Related to Figure 7;* Mean gene expression levels at each passage from transcriptomic data relating to Transforming Growth Factor β genes. ANOVA was used to test significant variation from P1 through to P15. No significant changes were observed

| Gene | p1 | p5 | p10 | p15 | ANOVA P value |
|------|-----|-----|-----|-----|---------------|
| CTGF | 12.93129068 | 13.11114526 | 12.95890603 | 13.78806378 | 0.236277103 |
| PTGFR | 14.11406285 | 13.7972693 | 13.15233422 | 12.80056628 | 0.185256607 |
| PTGFRN | 10.48101411 | 10.51169203 | 10.6445946 | 10.72228582 | 0.98685427 |
| TGFA | 2.720032823 | 2.436113251 | 1.972078689 | 2.588237911 | 0.499795593 |
| TGFB1 | 6.727452624 | 6.543144794 | 6.651820715 | 6.426532051 | 0.820938007 |
| TGFB1I1 | 8.20127917 | 8.092501175 | 7.928413536 | 7.684186941 | 0.292599591 |
| TGFB2 | 9.038249337 | 9.530277765 | 9.524899648 | 10.15981589 | 0.366663633 |
| TGFB3 | 10.16573317 | 9.227449618 | 8.585776596 | 8.340849151 | 0.123002756 |
| TGFBI | 14.23009913 | 14.17688001 | 14.05364426 | 13.94196898 | 0.928362048 |
| TGFBR1 | 12.59078859 | 12.81077217 | 13.11977441 | 13.19717954 | 0.642588238 |
| TGFBR2 | 12.53651792 | 12.61765041 | 12.9401318 | 13.29719118 | 0.431966177 |
| TGFBR3 | 10.54188845 | 10.66198291 | 10.78132306 | 10.94399838 | 0.86251197 |
| TGFBR3L | 1.920506931 | 1.812601554 | 1.934937741 | 1.720610947 | 0.776172143 |
| TGFBRAP1 | 9.487183102 | 9.465681952 | 9.654275737 | 9.419263045 | 0.572928938 |

Table S5. *Related to Figure 7;* Mean protein expression levels at each passage from proteomic data for proteins abundantly expressed at all passages

| Protein | p1 | p5 | p10 | p15 | Mean Expression |
|---------|-----|-----|-----|-----|------------------|
| Metalloproteinase Inhibitor 1 (Tissue Inhibitor of Metalloproteinase 1; TIMP1) | $5.47 \times 10^9$ | $6.45 \times 10^9$ | $6.35 \times 10^9$ | $6.99 \times 10^9$ | $6.31 \times 10^9$ |
| Fibulin 1 | $1.95 \times 10^9$ | $1.50 \times 10^9$ | $2.30 \times 10^9$ | $2.26 \times 10^9$ | $2.00 \times 10^9$ |
| Insulin-like growth factor binding protein 7 (IGFBP7) | $9.03 \times 10^8$ | $1.61 \times 10^9$ | $1.91 \times 10^9$ | $2.05 \times 10^9$ | $1.62 \times 10^9$ |
| Prelamin-A/C | $1.46 \times 10^9$ | $1.47 \times 10^9$ | $1.50 \times 10^9$ | $1.48 \times 10^9$ | $1.48 \times 10^9$ |
| Myosin-9 | $9.83 \times 10^8$ | $9.39 \times 10^8$ | $9.10 \times 10^8$ | $9.49 \times 10^8$ | $9.45 \times 10^8$ |
| Complement C1s subcomponent | $6.57 \times 10^8$ | $7.22 \times 10^8$ | $1.15 \times 10^8$ | $1.12 \times 10^8$ | $9.12 \times 10^8$ |
| Thrombospondin-1 | $4.78 \times 10^8$ | $6.48 \times 10^8$ | $6.58 \times 10^8$ | $9.02 \times 10^8$ | $6.71 \times 10^8$ |
| Filamin B | $5.18 \times 10^8$ | $4.59 \times 10^8$ | $4.83 \times 10^8$ | $6.05 \times 10^8$ | $5.16 \times 10^8$ |
| Glia-derived nexin | $2.08 \times 10^8$ | $3.38 \times 10^8$ | $3.51 \times 10^8$ | $6.31 \times 10^8$ | $3.82 \times 10^8$ |

**Claims**

1.  A method of selecting an MSC-derived cell population for therapeutic use, the method comprising:

    • assessing levels of MMP13 gene expression and TIMP-1 protein secretion by a cell population, and
    • selecting a cell population for use in a therapeutic application that does not require a capacity for phenotypic differentiation if the cell population exhibits low levels of MMP13 gene expression defined by an average level of MMP13 expression relative to a housekeeping gene that is half or less than half of said relative expression by freshly isolated MSCs, and high levels of TIMP-1 protein secretion defined by an average secretion of 100ng/ml or more of TIMP-1 from cells initially plated at a density of 2.25 million cells/well in a 24 hour period, wherein the therapeutic application that does not require a capacity for phenotypic differentiation is selected from the group consisting of: promoting tissue repair via trophic activity; and promotion of therapeutic immunosuppression.

2.  A method according to claim 1, wherein the cell population is derived from MSCs by passaging of MSCs.

3.  A pharmaceutical composition comprising a population of cells enriched for an MSC-derived cell population , wherein said MSC-derived cell population is :

    • CD90$^+$; CD105$^+$; CD45$^-$
    • is **characterized by** a high TIMP-1 secretion defined by an average secretion of 100ng/ml or more of TIMP-1 from cells initially plated at a density of 2.25 million cells/well in a 24 hour period,
    • and a low MMP13 gene expression defined by an average level of MMP13 expression relative to a housekeeping gene that is half or less than half of said relative expression by freshly isolated MSCs, and Has trophic activity,
    • wherein the population of cells enriched for an MSC-derived cell population comprises at least 70% of cells having the recited marker profile,

    and a pharmaceutically acceptable excipient.

4.  The pharmaceutical composition according to claim 3, wherein the population of cells enriched for an MSC-derived cell population comprises at least 80% of cells that are positive for CD90 and CD105, and 10% or less of cells that are positive for CD45.

5.  The pharmaceutical composition according to claim 3, that is substantially free from cells that are: CD90$^+$; CD105$^+$; CD45$^-$; and have high TIMP-1 secretion and high MMP13 gene expression.

6.  The pharmaceutical composition according to claim 5, that is free from cells that are: CD90$^+$; CD105$^+$; CD45$^-$; and have high TIMP-1 secretion and high MMP13 gene expression.

**Patentansprüche**

1.  Verfahren zum Auswählen einer aus MSC stammenden Zellpopulation zur therapeutischen Verwendung, das Verfahren umfassend:

    • Bewerten von Niveaus von MMP13-Genexpression und TIMP-1-Proteinsekretion durch eine Zellpopulation, und
    • Auswählen einer Zellpopulation zur Verwendung in einer therapeutischen Anwendung, die keine Fähigkeit zur phänotypischen Differenzierung erfordert, wenn die Zellpopulation niedrige Niveaus von MMP13-Genexpression, definiert durch ein durchschnittliches Niveau von MMP13-Expression im Verhältnis zu einem Haushalsgen, das die Hälfte oder weniger als die Hälfte der relativen Expression durch frisch isolierte MSCs ist, und hohe Niveaus von TIMP-1-Proteinsekretion, definiert durch eine durchschnittliche Sekretion von 100 ng/ml oder mehr TIMP-1 aus Zellen, die ursprünglich in einer Dichte von 2,25 Millionen Zellen/Well in einem Zeitraum von 24 Stunden plattiert wurden, zeigt, wobei die therapeutische Anwendung, die keine Fähigkeit zur phänotypischen Differenzierung erfordert, ausgewählt ist aus der Gruppe, bestehend aus: Fördern von Gewebereparatur durch trophische Aktivität; und Fördern von therapeutischer Immunsuppression.

2.  Verfahren nach Anspruch 1, wobei die Zellpopulation aus MSCs durch Passagieren von MSCs stammen.

**3.** Pharmazeutische Zusammensetzung, umfassend eine Zellpopulation, die für eine aus MSC stammende Zellpopulation angereichert ist, wobei die von MSC stammende Zellpopulation wie folgt ist:

- CD90⁺; CD105⁺; CD45⁻
- **gekennzeichnet durch** eine hohe TIMP-1-Sekretion, definiert durch eine durchschnittliche Sekretion von 100 ng/ml oder mehr TIMP-1 aus Zellen, die anfänglich in einer Dichte von 2,25 Millionen Zellen/Well in einem Zeitraum von 24 Stunden plattiert werden,
- und eine niedrige MMP13-Genexpression, definiert durch ein durchschnittliches Niveau von MMP13-Expression im Verhältnis zu einem Haushalsgen, das die Hälfte oder weniger als die Hälfte der relativen Expression durch frisch isolierte MSCs ist, und trophische Aktivität aufweist,
- wobei die Zellpopulation, die für eine aus MSC stammende Zellpopulation angereichert ist, umfasst, dass mindestens 70 % der Zellen das angegebene Markerprofil aufweisen,

und einen pharmazeutisch annehmbaren Hilfsstoff.

**4.** Pharmazeutische Zusammensetzung nach Anspruch 3, wobei die Zellpopulation, die für eine aus MSC stammende Zellpopulation angereichert ist, umfasst, dass mindestens 80 % der Zellen positiv für CD90 und CD105 sind, und 10 % oder weniger der Zellen positiv für CD45 sind.

**5.** Pharmazeutische Zusammensetzung nach Anspruch 3, die im Wesentlichen frei von Zellen ist, die wie folgt sind: CD90⁺; CD105⁺; CD45⁻; und die eine hohe TIMP-1-Sekretion und eine hohe MMP13-Genexpression aufweisen.

**6.** Pharmazeutische Zusammensetzung nach Anspruch 5, die frei von Zellen ist, die wie folgt sind: CD90⁺; CD105⁺; CD45⁻; und die eine hohe TIMP-1-Sekretion und eine hohe MMP13-Genexpression aufweisen.

**Revendications**

**1.** Procédé de sélection d'une population de cellules dérivée de CSM pour un usage thérapeutique, le procédé comprenant :

- l'évaluation des niveaux d'expression du gène MMP13 et de sécrétion de la protéine TIMP-1 par une population de cellules, et
- la sélection d'une population de cellules pour une utilisation dans une application thérapeutique qui ne nécessite pas de capacité de différenciation phénotypique si la population de cellules présente de faibles niveaux d'expression du gène MMP13 définis par un niveau moyen d'expression de MMP13 par rapport à un gène domestique qui est la moitié ou moins de la moitié de ladite expression relative par des CSM fraîchement isolées, et des niveaux élevés de sécrétion de protéine TIMP-1 définis par une sécrétion moyenne supérieure ou égale à 100 ng/ml de TIMP-1 à partir de cellules initialement plaquées à une densité de 2,25 millions de cellules/puits sur une période de 24 heures, ladite application thérapeutique qui ne nécessite pas de capacité de différenciation phénotypique étant choisie dans le groupe constitué par : la promotion de la réparation tissulaire par l'intermédiaire d'une activité trophique ; et la promotion de l'immunosuppression thérapeutique.

**2.** Procédé selon la revendication 1, ladite population de cellules étant dérivée de CSM par passage de CSM.

**3.** Composition pharmaceutique comprenant une population de cellules enrichie pour une population de cellules dérivées de CSM, ladite population de cellules dérivées de CSM étant :

- CD90⁺ ; CD105⁺ ; CD45⁻

- étant **caractérisée par** une sécrétion élevée de TIMP-1 définie par une sécrétion moyenne supérieure ou égale à 100 ng/ml de TIMP-1 à partir de cellules initialement plaquées à une densité de 2,25 millions de cellules/puits sur une période de 24 heures,

- et une faible expression du gène MMP13 définie par un niveau moyen d'expression de MMP13 par rapport à un gène domestique qui est la moitié ou moins de la moitié de ladite expression relative par des CSM fraîchement isolées, et présente une activité trophique,
- ladite population de cellules enrichie pour une population de cellules dérivées de CSM comprenant au moins 70

29

% de cellules présentant le profil de marqueur cité,

et un excipient pharmaceutiquement acceptable.

4. Composition pharmaceutique selon la revendication 3, ladite population de cellules enrichie pour une population de cellules dérivées de CSM comprenant au moins 80 % de cellules qui sont positives pour CD90 et CD105, et 10 % ou moins de cellules qui sont positives pour CD45.

5. Composition pharmaceutique selon la revendication 3, qui est sensiblement exempte de cellules qui sont : CD90$^+$; CD105$^+$; CD45$^-$ ; et présentent une sécrétion élevée de TIMP-1 et une expression élevée du gène MMP13.

6. Composition pharmaceutique selon la revendication 5, qui est exempte de cellules qui sont : CD90$^+$; CD105$^+$; CD45$^-$; et présentent une sécrétion élevée de TIMP-1 et une expression élevée du gène MMP13.

Figure 1

EP 4 038 179 B1

Figure 2

Figure 3

Figure 4

Figure 5

Figure 6

Figure 7

Figure 8- related to Figure 3; guidance images for osteogenesis scoring system. Each experimental slide was scored as – (see CTL, controls); +; ++; +++ or ++++ by two independent observers.

Figure 9 - relationship between osteogenic scores and cumulative population doublings or passage number.

EP 4 038 179 B1

Figure 10 - guidance images for adipogenesis scoring system. Each experimental slide was scored as – (see CTL, controls); +; ++; +++ or ++++ by two independent observers.

Figure 11 – related to Figure 3; relationship between osteogenic scores and cumulative population doublings or passage number.

Figure 12 — related to Figures 5 and 6; ingenuity analysis of master regulator genes

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8431162 B2, ZUBA-SURMA EWA K [US]; DAWN BUDDHADEB [US]
- WO 2013067038 A1, NEOSTEM INC [US]; MARASCO WAYNE [US]
- WO 2019035668 A2, SUNGKWANG MEDICAL FOUND)

**Non-patent literature cited in the description**

- **YUEH-HSUN KEVIN YANG et al.** Changes in phenotype and differentiation potential of human mesenchymal stem cells aging in vitro. *STEM CELL RESEARCH & THERAPY*, 11 May 2018, vol. 9 (1)
- **VICTORIA CHAPMAN et al.** Therapeutic Benefit for Late, but Not Early, Passage Mesenchymal Stem Cells on Pain Behaviour in an Animal Model of Osteoarthritis. *STEM CELLS INTERNATIONAL*, 01 January 2017, vol. 2017, ISSN 1687-966X, 1-11
- **MIQI WANG et al.** Trophic stimulation of articular chondrocytes by late-passage mesenchymal stem cells in coculture: LATE-PASSAGE MESENCHYMAL STEM CELL COCULTURE. *JOURNAL OF ORTHOPAEDIC RESEARCH*, 29 August 2013, vol. 31, ISSN 0736-0266, 1936-1942
- The enhancement of chondrogenic differentiation of human mesenchymal stem cells by enzymatically regulated RGD functionalities. **SALINAS et al.** BIOMATERIALS. ELSEVIER, 04 March 2008, vol. 29, 2370-2377
- **WHITEHOUSE et al.** *Stem Cells Translation Medicine*, 2017, vol. 6, 1237-1248
- Remington's Pharmaceutical Sciences. 1980
- Remington: The Science and Practice of Pharmacy. Lippincott Williams & Wilkins, 01 May 2005